# EUROPEAN PATENT APPLICATION

(11) **EP 3 699 596 A1**
(43) Date of publication of application: **26.08.2020**
(21) Application number: 19158645.2
(22) Date of filing: 21.02.2019
(51) Int. Cl.: G01N 33/68

(54) **METHOD FOR THE DIAGNOSIS OF MACCE IN PATIENTS WHO UNDERWENT GASTROINTESTINAL SURGERY**

(71) Applicant: B.R.A.H.M.S GmbH, 16761 Hennigsdorf (DE); Linköping University Hospital, 581 83 Linköping (SE)
(72) Inventor: WILSON, Darius, 79539 Lörrach (DE); CHEW, Michelle, 581 85 Linköping (SE); ANDERSSON, Henrik, 581 85 Linköping (SE)
(74) Representative: Heidler, Philipp

(57) **Abstract**

The invention pertains to a method for the prognosis and/or risk assessment and/or diagnosis of a major adverse cardio- or cerebrovascular event (MACCE) in a patient who underwent gastrointestinal surgery, the method comprising the steps of: i) providing a sample of a bodily fluid from said patient, ii) determining in said sample the level of a biomarker selected from the group consisting of copeptin, troponin and brain natriuretic peptide (BNP), iii) determining at least one additional parameter of said patient, iv) combining the biomarker level determined in step ii) and the additional parameter determined in step iii) into a combined assessment, and v) correlating the combined assessment to said at least one of prognosis, risk assessment and diagnosis of a MACCE in said patient. The invention further pertains to a kit for carrying out the method, a computer and a computer program product comprising computer-executable code being configured to carry out steps iv) and/or v) of the method of the invention.

SEQUENCE LISTING

<110> B.R.A.H.M.S GmbH
      Linköping University Hospital

<120> METHOD FOR THE DIAGNOSIS OF MACCE IN PATIENTS WHO UNDERWENT
GASTROINTESTINAL SURGERY

<130> B.P 1694 EP

<160> 27

<170> BiSSAP 1.3.6

<210> 1
<211> 164
<212> PRT
<213> Homo sapiens


<220>
<223> amino acid sequence of pre-pro-AVP

<400> 1


<210> 2
<211> 145
<212> PRT
<213> Homo sapiens


<220>
<223> amino acid sequence of pro-AVP

<400> 2
Cys Tyr Phe Gln Asn Cys Pro Arg Gly Gly Lys Arg Ala Met Ser Asp
1               5                   10                  15
Leu Glu Leu Arg Gln Cys Leu Pro Cys Gly Pro Gly Gly Lys Gly Arg
            20                  25                  30
Cys Phe Gly Pro Ser Ile Cys Cys Ala Asp Glu Leu Gly Cys Phe Val
        35                  40                  45

Gly Thr Ala Glu Ala Leu Arg Cys Gln Glu Glu Asn Tyr Leu Pro Ser
    50                  55                  60
Pro Cys Gln Ser Gly Gln Lys Ala Cys Gly Ser Gly Gly Arg Cys Ala
65                  70                  75                  80
Ala Phe Gly Val Cys Cys Asn Asp Glu Ser Cys Val Thr Glu Pro Glu
                85                  90                  95
Cys Arg Glu Gly Phe His Arg Arg Ala Arg Ala Ser Asp Arg Ser Asn
            100                 105                 110
Ala Thr Gln Leu Asp Gly Pro Ala Gly Ala Leu Leu Leu Arg Leu Val
        115                 120                 125
Gln Leu Ala Gly Ala Pro Glu Pro Phe Glu Pro Ala Gln Pro Asp Ala
    130                 135                 140
Tyr
145

<210> 3
<211> 39
<212> PRT
<213> Homo sapiens

<220>
<223> amino acid sequence of CT-pre-proAVP or copeptin

<400> 3
Ala Ser Asp Arg Ser Asn Ala Thr Gln Leu Asp Gly Pro Ala Gly Ala
1               5                   10                  15
Leu Leu Leu Arg Leu Val Gln Leu Ala Gly Ala Pro Glu Pro Phe Glu
            20                  25                  30
Pro Ala Gln Pro Asp Ala Tyr
        35

<210> 4
<211> 93
<212> PRT
<213> Homo sapiens

<220>
<223> amino acid sequence of Neurophysin II

<400> 4
Ala Met Ser Asp Leu Glu Leu Arg Gln Cys Leu Pro Cys Gly Pro Gly
1               5                   10                  15
Gly Lys Gly Arg Cys Phe Gly Pro Ser Ile Cys Cys Ala Asp Glu Leu
            20                  25                  30
Gly Cys Phe Val Gly Thr Ala Glu Ala Leu Arg Cys Gln Glu Glu Asn
        35                  40                  45
Tyr Leu Pro Ser Pro Cys Gln Ser Gly Gln Lys Ala Cys Gly Ser Gly
    50                  55                  60
Gly Arg Cys Ala Ala Phe Gly Val Cys Cys Asn Asp Glu Ser Cys Val
65                  70                  75                  80
Thr Glu Pro Glu Cys Arg Glu Gly Phe His Arg Arg Ala
                85                  90

<210> 5
<211> 288
<212> PRT
<213> Homo sapiens

<220>
<223> amino acid sequence of cTnT (Isoform-6)

<400> 5

<210> 6
<211> 210
<212> PRT
<213> Homo sapiens

<220>
<223> amino acid sequence of cTnI

<400> 6

<210> 7
<211> 161
<212> PRT
<213> Homo sapiens


<220>
<223> amino acid sequence of TnC

<400> 7
Met Asp Asp Ile Tyr Lys Ala Ala Val Glu Gln Leu Thr Glu Glu Gln
1               5                   10                  15
Lys Asn Glu Phe Lys Ala Ala Phe Asp Ile Phe Val Leu Gly Ala Glu
            20                  25                  30
Asp Gly Cys Ile Ser Thr Lys Glu Leu Gly Lys Val Met Arg Met Leu
        35                  40                  45
Gly Gln Asn Pro Thr Pro Glu Glu Leu Gln Glu Met Ile Asp Glu Val
    50                  55                  60
Asp Glu Asp Gly Ser Gly Thr Val Asp Phe Asp Glu Phe Leu Val Met
65                  70                  75                  80
Met Val Arg Cys Met Lys Asp Asp Ser Lys Gly Lys Ser Glu Glu Glu
                85                  90                  95
Leu Ser Asp Leu Phe Arg Met Phe Asp Lys Asn Ala Asp Gly Tyr Ile
            100                 105                 110
Asp Leu Asp Glu Leu Lys Ile Met Leu Gln Ala Thr Gly Glu Thr Ile
        115                 120                 125
Thr Glu Asp Asp Ile Glu Glu Leu Met Lys Asp Gly Asp Lys Asn Asn
    130                 135                 140
Asp Gly Arg Ile Asp Tyr Asp Glu Phe Leu Glu Phe Met Lys Gly Val
145                 150                 155                 160
Glu


<210> 8
<211> 134
<212> PRT
<213> Homo sapiens


<220>
<223> amino acid sequence of pre-pro-BNP

<400> 8
Met Asp Pro Gln Thr Ala Pro Ser Arg Ala Leu Leu Leu Leu Leu Phe
1               5                   10                  15
Leu His Leu Ala Phe Leu Gly Gly Arg Ser His Pro Leu Gly Ser Pro

20                      25                      30
Gly Ser Ala Ser Asp Leu Glu Thr Ser Gly Leu Gln Glu Gln Arg Asn
        35                      40                      45
His Leu Gln Gly Lys Leu Ser Glu Leu Gln Val Glu Gln Thr Ser Leu
    50                      55                      60
Glu Pro Leu Gln Glu Ser Pro Arg Pro Thr Gly Val Trp Lys Ser Arg
65                      70                      75                      80
Glu Val Ala Thr Glu Gly Ile Arg Gly His Arg Lys Met Val Leu Tyr
                85                      90                      95
Thr Leu Arg Ala Pro Arg Ser Pro Lys Met Val Gln Gly Ser Gly Cys
            100                     105                     110
Phe Gly Arg Lys Met Asp Arg Ile Ser Ser Ser Ser Gly Leu Gly Cys
        115                     120                     125
Lys Val Leu Arg Arg His
    130

<210> 9
<211> 108
<212> PRT
<213> Homo sapiens

<220>
<223> amino acid sequence of pro-BNP

<400> 9
His Pro Leu Gly Ser Pro Gly Ser Ala Ser Asp Leu Glu Thr Ser Gly
1               5                   10                  15
Leu Gln Glu Gln Arg Asn His Leu Gln Gly Lys Leu Ser Glu Leu Gln
            20                  25                  30
Val Glu Gln Thr Ser Leu Glu Pro Leu Gln Glu Ser Pro Arg Pro Thr
        35                  40                  45
Gly Val Trp Lys Ser Arg Glu Val Ala Thr Glu Gly Ile Arg Gly His
    50                  55                  60
Arg Lys Met Val Leu Tyr Thr Leu Arg Ala Pro Arg Ser Pro Lys Met
65                  70                  75                  80
Val Gln Gly Ser Gly Cys Phe Gly Arg Lys Met Asp Arg Ile Ser Ser
                85                  90                  95
Ser Ser Gly Leu Gly Cys Lys Val Leu Arg Arg His
            100                 105

<210> 10
<211> 76
<212> PRT
<213> Homo sapiens

<220>
<223> amino acid sequence of NT-pro-BNP

<400> 10
His Pro Leu Gly Ser Pro Gly Ser Ala Ser Asp Leu Glu Thr Ser Gly
1               5                   10                  15
Leu Gln Glu Gln Arg Asn His Leu Gln Gly Lys Leu Ser Glu Leu Gln
            20                  25                  30
Val Glu Gln Thr Ser Leu Glu Pro Leu Gln Glu Ser Pro Arg Pro Thr
        35                  40                  45
Gly Val Trp Lys Ser Arg Glu Val Ala Thr Glu Gly Ile Arg Gly His
    50                  55                  60
Arg Lys Met Val Leu Tyr Thr Leu Arg Ala Pro Arg
65                  70                  75

<210> 11

<211> 32
<212> PRT
<213> Homo sapiens

<220>
<223> amino acid sequence of BNP

<400> 11
Ser Pro Lys Met Val Gln Gly Ser Gly Cys Phe Gly Arg Lys Met Asp
1               5                   10                  15
Arg Ile Ser Ser Ser Ser Gly Leu Gly Cys Lys Val Leu Arg Arg His
            20                  25                  30

<210> 12
<211> 185
<212> PRT
<213> Homo sapiens

<220>
<223> amino acid sequence of pre-pro-ADM

<400> 12
Met Lys Leu Val Ser Val Ala Leu Met Tyr Leu Gly Ser Leu Ala Phe
1               5                   10                  15
Leu Gly Ala Asp Thr Ala Arg Leu Asp Val Ala Ser Glu Phe Arg Lys
            20                  25                  30
Lys Trp Asn Lys Trp Ala Leu Ser Arg Gly Lys Arg Glu Leu Arg Met
        35                  40                  45
Ser Ser Ser Tyr Pro Thr Gly Leu Ala Asp Val Lys Ala Gly Pro Ala
    50                  55                  60
Gln Thr Leu Ile Arg Pro Gln Asp Met Lys Gly Ala Ser Arg Ser Pro
65                  70                  75                  80
Glu Asp Ser Ser Pro Asp Ala Ala Arg Ile Arg Val Lys Arg Tyr Arg
                85                  90                  95
Gln Ser Met Asn Asn Phe Gln Gly Leu Arg Ser Phe Gly Cys Arg Phe
            100                 105                 110
Gly Thr Cys Thr Val Gln Lys Leu Ala His Gln Ile Tyr Gln Phe Thr
        115                 120                 125
Asp Lys Asp Lys Asp Asn Val Ala Pro Arg Ser Lys Ile Ser Pro Gln
    130                 135                 140
Gly Tyr Gly Arg Arg Arg Arg Arg Ser Leu Pro Glu Ala Gly Pro Gly
145                 150                 155                 160
Arg Thr Leu Val Ser Ser Lys Pro Gln Ala His Gly Ala Pro Ala Pro
                165                 170                 175
Pro Ser Gly Ser Ala Pro His Phe Leu
            180                 185

<210> 13
<211> 164
<212> PRT
<213> Homo sapiens

<220>
<223> amino acid sequence of pro-ADM

<400> 13
Ala Arg Leu Asp Val Ala Ser Glu Phe Arg Lys Lys Trp Asn Lys Trp
1               5                   10                  15

Ala Leu Ser Arg Gly Lys Arg Glu Leu Arg Met Ser Ser Ser Tyr Pro
            20                  25                  30
Thr Gly Leu Ala Asp Val Lys Ala Gly Pro Ala Gln Thr Leu Ile Arg
        35                  40                  45
Pro Gln Asp Met Lys Gly Ala Ser Arg Ser Pro Glu Asp Ser Ser Pro
    50                  55                  60
Asp Ala Ala Arg Ile Arg Val Lys Arg Tyr Arg Gln Ser Met Asn Asn
65                  70                  75                  80
Phe Gln Gly Leu Arg Ser Phe Gly Cys Arg Phe Gly Thr Cys Thr Val
                85                  90                  95
Gln Lys Leu Ala His Gln Ile Tyr Gln Phe Thr Asp Lys Asp Lys Asp
            100                 105                 110
Asn Val Ala Pro Arg Ser Lys Ile Ser Pro Gln Gly Tyr Gly Arg Arg
        115                 120                 125
Arg Arg Arg Ser Leu Pro Glu Ala Gly Pro Gly Arg Thr Leu Val Ser
    130                 135                 140
Ser Lys Pro Gln Ala His Gly Ala Pro Ala Pro Pro Ser Gly Ser Ala
145                 150                 155                 160
Pro His Phe Leu

<210> 14
<211> 48
<212> PRT
<213> Homo sapiens

<220>
<223> amino acid sequence of MR-pro-ADM

<400> 14
Glu Leu Arg Met Ser Ser Ser Tyr Pro Thr Gly Leu Ala Asp Val Lys
1               5                   10                  15
Ala Gly Pro Ala Gln Thr Leu Ile Arg Pro Gln Asp Met Lys Gly Ala
            20                  25                  30
Ser Arg Ser Pro Glu Asp Ser Ser Pro Asp Ala Ala Arg Ile Arg Val
        35                  40                  45

<210> 15
<211> 212
<212> PRT
<213> Homo sapiens

<220>
<223> amino acid sequence of pre-pro-ET-1

<400> 15
Met Asp Tyr Leu Leu Met Ile Phe Ser Leu Leu Phe Val Ala Cys Gln
1               5                   10                  15
Gly Ala Pro Glu Thr Ala Val Leu Gly Ala Glu Leu Ser Ala Val Gly
            20                  25                  30
Glu Asn Gly Gly Glu Lys Pro Thr Pro Ser Pro Pro Trp Arg Leu Arg
        35                  40                  45
Arg Ser Lys Arg Cys Ser Cys Ser Ser Leu Met Asp Lys Glu Cys Val
    50                  55                  60
Tyr Phe Cys His Leu Asp Ile Ile Trp Val Asn Thr Pro Glu His Val
65                  70                  75                  80
Val Pro Tyr Gly Leu Gly Ser Pro Arg Ser Lys Arg Ala Leu Glu Asn
                85                  90                  95
Leu Leu Pro Thr Lys Ala Thr Asp Arg Glu Asn Arg Cys Gln Cys Ala

100                     105                     110
Ser Gln Lys Asp Lys Lys Cys Trp Asn Phe Cys Gln Ala Gly Lys Glu
        115                     120                     125
Leu Arg Ala Glu Asp Ile Met Glu Lys Asp Trp Asn Asn His Lys Lys
    130                     135                     140
Gly Lys Asp Cys Ser Lys Leu Gly Lys Lys Cys Ile Tyr Gln Gln Leu
145                     150                     155                     160
Val Arg Gly Arg Lys Ile Arg Arg Ser Ser Glu Glu His Leu Arg Gln
                165                     170                     175
Thr Arg Ser Glu Thr Met Arg Asn Ser Val Lys Ser Ser Phe His Asp
            180                     185                     190
Pro Lys Leu Lys Gly Lys Pro Ser Arg Glu Arg Tyr Val Thr His Asn
        195                     200                     205
Arg Ala His Trp
    210

<210> 16
<211> 195
<212> PRT
<213> Homo sapiens

<220>
<223> amino acid sequence of pro-ET-1

<400> 16
Ala Pro Glu Thr Ala Val Leu Gly Ala Glu Leu Ser Ala Val Gly Glu
1               5                   10                  15
Asn Gly Gly Glu Lys Pro Thr Pro Ser Pro Pro Trp Arg Leu Arg Arg
            20                  25                  30
Ser Lys Arg Cys Ser Cys Ser Ser Leu Met Asp Lys Glu Cys Val Tyr
        35                  40                  45
Phe Cys His Leu Asp Ile Ile Trp Val Asn Thr Pro Glu His Val Val
    50                  55                  60
Pro Tyr Gly Leu Gly Ser Pro Arg Ser Lys Arg Ala Leu Glu Asn Leu
65                  70                  75                  80
Leu Pro Thr Lys Ala Thr Asp Arg Glu Asn Arg Cys Gln Cys Ala Ser
                85                  90                  95
Gln Lys Asp Lys Lys Cys Trp Asn Phe Cys Gln Ala Gly Lys Glu Leu
            100                 105                 110
Arg Ala Glu Asp Ile Met Glu Lys Asp Trp Asn Asn His Lys Lys Gly
        115                 120                 125
Lys Asp Cys Ser Lys Leu Gly Lys Lys Cys Ile Tyr Gln Gln Leu Val
    130                 135                 140
Arg Gly Arg Lys Ile Arg Arg Ser Ser Glu Glu His Leu Arg Gln Thr
145                 150                 155                 160
Arg Ser Glu Thr Met Arg Asn Ser Val Lys Ser Ser Phe His Asp Pro
                165                 170                 175
Lys Leu Lys Gly Lys Pro Ser Arg Glu Arg Tyr Val Thr His Asn Arg
            180                 185                 190
Ala His Trp
        195

<210> 17
<211> 21
<212> PRT
<213> Homo sapiens

<220>
<223> amino acid sequence of pro-ET-1

<400> 17

Cys Ser Cys Ser Ser Leu Met Asp Lys Glu Cys Val Tyr Phe Cys His
1               5                   10                  15
Leu Asp Ile Ile Trp
            20

<210> 18
<211> 45
<212> PRT
<213> Homo sapiens


<220>
<223> amino acid sequence of CT-pro-ET-1

<400> 18
Arg Ser Ser Glu Glu His Leu Arg Gln Thr Arg Ser Glu Thr Met Arg
1               5                   10                  15
Asn Ser Val Lys Ser Ser Phe His Asp Pro Lys Leu Lys Gly Lys Pro
            20                  25                  30
Ser Arg Glu Arg Tyr Val Thr His Asn Arg Ala His Trp
        35                  40                  45

<210> 19
<211> 38
<212> PRT
<213> Homo sapiens


<220>
<223> amino acid sequence of Big-ET-1

<400> 19
Cys Ser Cys Ser Ser Leu Met Asp Lys Glu Cys Val Tyr Phe Cys His
1               5                   10                  15
Leu Asp Ile Ile Trp Val Asn Thr Pro Glu His Val Val Pro Tyr Gly
            20                  25                  30
Leu Gly Ser Pro Arg Ser
        35

<210> 20
<211> 116
<212> PRT
<213> Homo sapiens


<220>
<223> amino acid sequence of PCT

<400> 20
Ala Pro Phe Arg Ser Ala Leu Glu Ser Ser Pro Ala Asp Pro Ala Thr
1               5                   10                  15
Leu Ser Glu Asp Glu Ala Arg Leu Leu Leu Ala Ala Leu Val Gln Asp
            20                  25                  30
Tyr Val Gln Met Lys Ala Ser Glu Leu Glu Gln Glu Gln Glu Arg Glu
        35                  40                  45
Gly Ser Ser Leu Asp Ser Pro Arg Ser Lys Arg Cys Gly Asn Leu Ser
    50                  55                  60
Thr Cys Met Leu Gly Thr Tyr Thr Gln Asp Phe Asn Lys Phe His Thr
65                  70                  75                  80
Phe Pro Gln Thr Ala Ile Gly Val Gly Ala Pro Gly Lys Lys Arg Asp
                85                  90                  95
Met Ser Ser Asp Leu Glu Arg Asp His Arg Pro His Val Ser Met Pro
            100                 105                 110

Gln Asn Ala Asn
        115

<210> 21
<211> 153
<212> PRT
<213> Homo sapiens


<220>
<223> amino acid sequence of pre-pro-ANP

<400> 21

<210> 22
<211> 126
<212> PRT
<213> Homo sapiens


<220>
<223> amino acid sequence of pro-ANP

<400> 22
Asn Pro Met Tyr Asn Ala Val Ser Asn Ala Asp Leu Met Asp Phe Lys
1               5                   10                  15
Asn Leu Leu Asp His Leu Glu Glu Lys Met Pro Leu Glu Asp Glu Val
            20                  25                  30
Val Pro Pro Gln Val Leu Ser Glu Pro Asn Glu Glu Ala Gly Ala Ala
        35                  40                  45
Leu Ser Pro Leu Pro Glu Val Pro Pro Trp Thr Gly Glu Val Ser Pro
    50                  55                  60
Ala Gln Arg Asp Gly Gly Ala Leu Gly Arg Gly Pro Trp Asp Ser Ser
65                  70                  75                  80
Asp Arg Ser Ala Leu Leu Lys Ser Lys Leu Arg Ala Leu Leu Thr Ala
                85                  90                  95
Pro Arg Ser Leu Arg Arg Ser Ser Cys Phe Gly Gly Arg Met Asp Arg
            100                 105                 110
Ile Gly Ala Gln Ser Gly Leu Gly Cys Asn Ser Phe Arg Tyr
        115                 120                 125

<210> 23
<211> 98

<212> PRT
<213> Homo sapiens

<220>
<223> amino acid sequence of NT-proANP

<400> 23
Asn Pro Met Tyr Asn Ala Val Ser Asn Ala Asp Leu Met Asp Phe Lys
1               5                   10                  15
Asn Leu Leu Asp His Leu Glu Glu Lys Met Pro Leu Glu Asp Glu Val
            20                  25                  30
Val Pro Pro Gln Val Leu Ser Glu Pro Asn Glu Glu Ala Gly Ala Ala
        35                  40                  45
Leu Ser Pro Leu Pro Glu Val Pro Pro Trp Thr Gly Glu Val Ser Pro
    50                  55                  60
Ala Gln Arg Asp Gly Gly Ala Leu Gly Arg Gly Pro Trp Asp Ser Ser
65                  70                  75                  80
Asp Arg Ser Ala Leu Leu Lys Ser Lys Leu Arg Ala Leu Leu Thr Ala
                85                  90                  95
Pro Arg

<210> 24
<211> 38
<212> PRT
<213> Homo sapiens

<220>
<223> MR-proANP = amino acid sequence of amino acids 53-90 of proANP

<400> 24
Pro Glu Val Pro Pro Trp Thr Gly Glu Val Ser Pro Ala Gln Arg Asp
1               5                   10                  15
Gly Gly Ala Leu Gly Arg Gly Pro Trp Asp Ser Ser Asp Arg Ser Ala
            20                  25                  30
Leu Leu Lys Ser Lys Leu
        35

<210> 25
<211> 154
<212> PRT
<213> Homo sapiens

<220>
<223> Myoglobin

<400> 25
Met Gly Leu Ser Asp Gly Glu Trp Gln Leu Val Leu Asn Val Trp Gly
1               5                   10                  15
Lys Val Glu Ala Asp Ile Pro Gly His Gly Gln Glu Val Leu Ile Arg
            20                  25                  30
Leu Phe Lys Gly His Pro Glu Thr Leu Glu Lys Phe Asp Lys Phe Lys
        35                  40                  45
His Leu Lys Ser Glu Asp Glu Met Lys Ala Ser Glu Asp Leu Lys Lys
    50                  55                  60
His Gly Ala Thr Val Leu Thr Ala Leu Gly Gly Ile Leu Lys Lys Lys
65                  70                  75                  80
Gly His His Glu Ala Glu Ile Lys Pro Leu Ala Gln Ser His Ala Thr
                85                  90                  95
Lys His Lys Ile Pro Val Lys Tyr Leu Glu Phe Ile Ser Glu Cys Ile

                100                 105                 110
Ile Gln Val Leu Gln Ser Lys His Pro Gly Asp Phe Gly Ala Asp Ala
        115                 120                 125
Gln Gly Ala Met Asn Lys Ala Leu Glu Leu Phe Arg Lys Asp Met Ala
    130                 135                 140
Ser Asn Tyr Lys Glu Leu Gly Phe Gln Gly
145                 150

<210> 26
<211> 381
<212> PRT
<213> Homo sapiens

<220>
<223> Creatine kinase

<400> 26
Met Pro Phe Ser Asn Ser His Asn Ala Leu Lys Leu Arg Phe Pro Ala
1               5                   10                  15
Glu Asp Glu Phe Pro Asp Leu Ser Ala His Asn Asn His Met Ala Lys
            20                  25                  30
Val Leu Thr Pro Glu Leu Tyr Ala Glu Leu Arg Ala Lys Ser Thr Pro
        35                  40                  45
Ser Gly Phe Thr Leu Asp Asp Val Ile Gln Thr Gly Val Asp Asn Pro
    50                  55                  60
Gly His Pro Tyr Ile Met Thr Val Gly Cys Val Ala Gly Asp Glu Glu
65                  70                  75                  80
Ser Tyr Glu Val Phe Lys Asp Leu Phe Asp Pro Ile Ile Glu Asp Arg
                85                  90                  95
His Gly Gly Tyr Lys Pro Ser Asp Glu His Lys Thr Asp Leu Asn Pro
            100                 105                 110
Asp Asn Leu Gln Gly Gly Asp Asp Leu Asp Pro Asn Tyr Val Leu Ser
        115                 120                 125
Ser Arg Val Arg Thr Gly Arg Ser Ile Arg Gly Phe Cys Leu Pro Pro
    130                 135                 140
His Cys Ser Arg Gly Glu Arg Arg Ala Ile Glu Lys Leu Ala Val Glu
145                 150                 155                 160
Ala Leu Ser Ser Leu Asp Gly Asp Leu Ala Gly Arg Tyr Tyr Ala Leu
                165                 170                 175
Lys Ser Met Thr Glu Ala Glu Gln Gln Gln Leu Ile Asp Asp His Phe
            180                 185                 190
Leu Phe Asp Lys Pro Val Ser Pro Leu Leu Leu Ala Ser Gly Met Ala
        195                 200                 205
Arg Asp Trp Pro Asp Ala Arg Gly Ile Trp His Asn Asp Asn Lys Thr
    210                 215                 220
Phe Leu Val Trp Val Asn Glu Glu Asp His Leu Arg Val Ile Ser Met
225                 230                 235                 240
Gln Lys Gly Gly Asn Met Lys Glu Val Phe Thr Arg Phe Cys Thr Gly
                245                 250                 255
Leu Thr Gln Ile Glu Thr Leu Phe Lys Ser Lys Asp Tyr Glu Phe Met
            260                 265                 270
Trp Asn Pro His Leu Gly Tyr Ile Leu Thr Cys Pro Ser Asn Leu Gly
        275                 280                 285
Thr Gly Leu Arg Ala Gly Val His Ile Lys Leu Pro Asn Leu Gly Lys
    290                 295                 300
His Glu Lys Phe Ser Glu Val Leu Lys Arg Leu Arg Leu Gln Lys Arg
305                 310                 315                 320
Gly Thr Gly Gly Val Asp Thr Ala Ala Val Gly Gly Val Phe Asp Val
                325                 330                 335
Ser Asn Ala Asp Arg Leu Gly Phe Ser Glu Val Glu Leu Val Gln Met
            340                 345                 350
Val Val Asp Gly Val Lys Leu Leu Ile Glu Met Glu Gln Arg Leu Glu

                355                 360                 365
Gln Gly Gln Ala Ile Asp Asp Leu Met Pro Ala Gln Lys
    370                 375                 380

<210> 27
<211> 224
<212> PRT
<213> Homo sapiens


<220>
<223> C reactive Protein

<400> 27
Met Glu Lys Leu Leu Cys Phe Leu Val Leu Thr Ser Leu Ser His Ala
1               5                   10                  15
Phe Gly Gln Thr Asp Met Ser Arg Lys Ala Phe Val Phe Pro Lys Glu
            20                  25                  30
Ser Asp Thr Ser Tyr Val Ser Leu Lys Ala Pro Leu Thr Lys Pro Leu
        35                  40                  45
Lys Ala Phe Thr Val Cys Leu His Phe Tyr Thr Glu Leu Ser Ser Thr
    50                  55                  60
Arg Gly Tyr Ser Ile Phe Ser Tyr Ala Thr Lys Arg Gln Asp Asn Glu
65                  70                  75                  80
Ile Leu Ile Phe Trp Ser Lys Asp Ile Gly Tyr Ser Phe Thr Val Gly
                85                  90                  95
Gly Ser Glu Ile Leu Phe Glu Val Pro Glu Val Thr Val Ala Pro Val
            100                 105                 110
His Ile Cys Thr Ser Trp Glu Ser Ala Ser Gly Ile Val Glu Phe Trp
        115                 120                 125
Val Asp Gly Lys Pro Arg Val Arg Lys Ser Leu Lys Lys Gly Tyr Thr
    130                 135                 140
Val Gly Ala Glu Ala Ser Ile Ile Leu Gly Gln Glu Gln Asp Ser Phe
145                 150                 155                 160
Gly Gly Asn Phe Glu Gly Ser Gln Ser Leu Val Gly Asp Ile Gly Asn
                165                 170                 175
Val Asn Met Trp Asp Phe Val Leu Ser Pro Asp Glu Ile Asn Thr Ile
            180                 185                 190
Tyr Leu Gly Gly Pro Phe Ser Pro Asn Val Leu Asn Trp Arg Ala Leu
        195                 200                 205
Lys Tyr Glu Val Gln Gly Glu Val Phe Thr Lys Pro Gln Leu Trp Pro
    210                 215                 220

## Description

The present invention pertains to a method for the prognosis and diagnosis of a major adverse cardio- or cerebrovascular event (MACCE) in a patient who has undergone major gastrointestinal surgery. The invention also pertains to a kit for carrying out said method.

Worldwide, more than 200 million adults aged 45 or older undergo major noncardiac surgery each year [Weiser et al Lancet 2015*.*,]. Out of these, millions die from complications within 30 days [Botto et al., Anesthesiology, 120(3), pp. 564-578. doi: 10.1097/ALN.0000000000000113] and a significant proportion of patients suffer from major cardiac complications during the first year after surgery [Ekeloef et al., British Journal of Anaesthesia, 117(5), pp. 559-568. doi: 10.1093/bja/aew321]. The prime cause of postoperative mortality is cardiac complications due to ischemic injury of the myocardium, causing cell damage and uncontrolled cell death [Botto et al., Anesthesiology, 120(3), pp. 564-578. doi: 10.1097/ALN.0000000000000113; Sessler and Khanna; Intensive Care Med (2018) 44:811-822, https://doi.org/10.1007/s00134-018-5224-7; Landesberg et al., Circulation, 119(22), pp. 2936-2944. doi: 10.1161/CIRCULATIONAHA.108.828228]*.* In addition to the number of deaths, postoperative cardiac injuries result in a substantial number of postoperative complications prolonging recovery and hospitalisation, which results in increased patient suffering and medical costs [Devereaux and Sessler, New England journal of medicine, p. 2258, 2015].

MACCE comprise the most common causes of serious perioperative morbidity and mortality and depending on the population studied the incidence ranges between 1-7%. 10% of patients who develop MACCE die during their hospital stay. Several independent preoperative and intraoperative risk factors for MACCE have been identified, e.g. coronary artery disease, chronic congestive heart failure, intraoperative hypotension, and blood transfusion. It has been suggested that these are potentially able to stratify patients according to their risk of MACCE. Current risk stratification systems however have only limited sensitivity and specificity. Thus, further research is needed to identify better prediction models and risk stratification scores [Sabaté et al., British Journal of Anaesthesia, p. 879, 2011].

Perioperative myocardial infarction (PMI) is a common cardiovascular complication and is associated with poor outcomes. 12% of patients with PMI die within 30 days and most deaths occur within 48 hours [Hanson et al., Catheterization and Cardiovascular Interventions, 82(4), pp. 622-628. doi: 10.1002/ccd.24626]. However, 58.2% of patients with ischemic myocardial injury do not fulfill the universal definition for myocardial infarction (MI) even though these cardiac injuries are independently associated with 30-day mortality [Botto et al., Anesthesiology, 120(3), pp. 564-578. doi: 10.1097/ALN.0000000000000113; Noordzij et al., British Journal of Anaesthesia, 114(6), pp. 909-918. doi: 10.1093/bja/aev027, 2015].

The third universal definition of MI is necrosis of the myocardium caused by prolonged ischemia and is a common cause of death and disability. This definition was set by the European Society of Cardiology, American College of Cardiology Foundation, American Heart Association, and World Heart Federation. Two distinct mechanisms may cause MI, both of which are seen after noncardiac surgery. A type I MI is caused by an acute coronary syndrome (ACS); a rupture, ulceration, erosion or dissection of an unstable atherosclerotic plaque in one or multiple coronary vessels with subsequent intraluminal thrombosis leading to reduced myocardial blood flow. A type II MI is caused by a prolonged imbalance between myocardial oxygen supply and demand and may arise as a consequence of conditions, such as cardiac arrhythmia, anemia, respiratory failure, hypotension, hypertension or by direct toxic effects by high levels of endogenous or exogenous circulating cortisol and catecholamines [Landesberg et al., Circulation, 119(22), pp. 2936-2944. doi: 10.1161/CIRCULA-TIONAHA.108.828228*;* Thygesen et al., Circulation, 126(16), p. 2020. doi: 10.1161/CIR.0b013e31826e1058].

The diagnostic criteria of MI consist of a new elevation of the cardiac biomarker troponin, greater than 3 times the upper level of the reference range in the setting of suspected myocardial ischemia with either an ischemic symptom or an ischemic electrocardiographic (ECG) finding. The necrosis of myocardial cells due to ischemia requires at least 2-4 hours depending on individual variation in e.g. collateral circulation to the ischemic zone, the sensitivity of the myocytes to ischemia, prior myocardial necrosis and coronary arterial size and occlusion. These variations may also impact ECG manifestation of ischemia and therefore an ECG should always be compared to a prior one when available. Acute or evolving ECG abnormalities can potentially allow the clinician to identify the infarction location and the amount of myocardium at risk. The earliest manifestations of myocardial ischemia are typically changes in T-wave and ST-segment. More profound shifts or inversion involving multiple leads/myocardial areas is associated with a greater degree of myocardial ischemia and a worse prognosis. An ECG by itself is often insufficient to diagnose myocardial ischemia and infarction since the ischemic patterns may be caused by other conditions, e.g. ST deviation in acute pericarditis, left bundle branch block (LBBB), left ventricular hypertrophy (LVH) and abnormal Q-waves in e.g. amyloidosis of the heart, acute cor pulmonale and hyperkalemia [Thygesen et al., Circulation, 126(16), p. 2020. doi: 10.1161/CIR.0b013e31826e1058].

The ischemic symptoms of MI include various combinations of nonspecific symptoms, such as chest-, upper extremity-, mandibular pain and dyspnea. Symptoms often last >20 minutes, are diffuse, not localized, positional or affected by movement. MI-patients can also be asymptomatic, this is most common in women, the elderly, diabetics, in post-operative and critically ill patients [Thygesen et al., Circulation, 126(16), p. 2020. doi: 10.1161/CIR.0b013e31826e1058]. Most postoperative myocardial injuries occur within 48 hours after noncardiac surgery. A possible explanation for the lack of symptoms during this period is that the patients usually receive analgesic medications that mask the symptoms. This is thought to partly explain why 65% of the PMI are asymptomatic [Devereaux and Sessler, New England journal of medicine, p. 2258, 2015*;* Devereaux et al., JAMA, 317(16), pp. 1642-1651. doi: 10.1001/jama.2017.4360, 2017].

Myocardial injury after noncardiac surgery (MINS), is a prognostically relevant myocardial injury due to ischemia occurring in 8% of surgical patients [Botto et al., Anesthesiology, 120(3), pp. 564-578. doi: 10.1097/ALN.0000000000000113]. 10% of patients suffering from MINS die within 30 days, MINS-patients also have a greater 1-year mortality as well as beyond 1 year and a greater incidence of nonlethal cardiac arrest, congestive heart failure and stroke [Botto et al., Anesthesiology, 120(3), pp. 564-578. doi: 10.1097/ALN.0000000000000113; Mauermann et al., Current Opinion in Anaesthesiology, 29(3), pp. 403-412. doi: 10.1097/ACO.0000000000000336]*.* Furthermore, the regional myocardial injury can stun the myocardium, resulting in diastolic dysfunction and reduced cardiac output resulting in reduced peripheral blood supply, impaired healing of tissue and increased risk of wound infection [Noordzij et al., British Journal of Anaesthesia, 114(6), pp. 909-918. doi: 10.1093/bja/aev027, 2015].

The diagnostic criteria for MINS is a postoperative troponin elevation judged to be due to myocardial ischemia (no evidence of nonischemic etiology) with or without ischemic symptoms. MINS does not include perioperative myocardial damage originating from another etiology than ischemia, e.g. sepsis or pulmonary embolism. The postoperative thresholds for MINS depend on the type of troponin assay. For 5^{th} generation cTnT, the threshold is >20 ng/L with an increase from baseline of at least 5 ng/L or >65 ng/L [Sessler and Khanna; Intensive Care Med (2018) 44:811-822, https://doi.org/10.1007/s00134-018-5224-7]. The criteria for MINS cover a wider spectrum of myocardial injury than MI, from reversible myocardial injury to necrosis. This broader concept comprises several manifestations of myocardial injury and makes MINS a more applicable term for surgical patients and lowers the risk of underestimating the extent of myocardial damage seen after noncardiac surgery [Mauermann et al., Current Opinion in Anaesthesiology, 29(3), pp. 403-412. doi: 10.1097/ACO.0000000000000336].

93.6% of MINS occur within 3 days of surgery, MINS patients will have mild troponin elevation and often lack ischemic features [Noordzij et al., British Journal of Anaesthesia, 114(6), pp. 909-918. doi: 10.1093/bja/aev027, 2015]. 84% of patients suffering from MINS do not experience ischemic symptoms and only 34.9% will show signs of ischemia on ECG [Botto et al., Anesthesiology, 120(3), pp. 564-578. doi: 10.1097/ALN.0000000000000113]. These findings indicate that a reliable diagnosis of perioperative myocardial injury, in most cases, is only possible by continuous postoperative troponin measurements. Otherwise, it is reported that 93.1% of MINS and 68% of PMI may go undetected [Devereaux et al., JAMA, 317(16), pp. 1642-1651. doi: 10.1001/jama.2017.4360, 2017].

The mechanism of MINS remains unclear, but is suggested to be similar to the mechanisms behind PMI [Mauermann et al., Current Opinion in Anaesthesiology, 29(3), pp. 403-412. doi: 10.1097/ACO.0000000000000336]. Several trials have been conducted trying to counteract MINS, but there are still no known methods safely and efficiently preventing the myocardial injuries [Sessler and Khanna; Intensive Care Med (2018) 44:811-822, https://doi.org/10.1007//s00134-018-5224-7]. One example, the POISE-study, a large randomized trial administering beta blockers perioperative and postoperative, managed to reduce the occurrence of major cardiovascular events by 30% but at the same time increased the incidence of serious hypotension, stroke and mortality [Devereaux et al., Lancet, 371(9627), pp. 1839-1847. doi: 10.1016/S0140-6736(08)60601-7].

Previous studies investigated the use of biomarkers in predicting postoperative outcomes in patients. For example, a correlation of the expression of arginine vasopressin (AVP) and copeptin to the severity of systemic inflammatory response syndrome (SIRS) with and without shock in patients after non-cardiac surgery could be shown [Jochberger et al., SHOCK, Vol. 31, No. 2, pp. 132-138, 2009]. Copeptin rises even higher in SIRS patients after cardiac surgery [Jochberger et al., J Clin Endo-crinol Metab 91:4381-4386, 2006]. Another study showed that copeptin is increased in patients with inflammatory bowel disease (IBD) and even more increased in IBD patients who underwent bowel resections [Ohlsson and Melander, Drug Target Insights 2015:9, 21-27 DOI: 10.4137/DTI.S26589]. Copeptin as a general stress biomarker is known to rise in response to surgical trauma, however, no difference between minimally invasive and conventional surgical techniques could be found [Netto et al., Acta Chirurgica Belgica, DOI: 10.1080/00015458.2018.1482698, 2018]. Pre-operative copeptin and NT-proBNP levels have been investigated to improve risk stratification for patients undergoing major vascular surgery, showing that increased biomarker levels are predictive of outcome in this patient group [Jarai et al., Am J Cardiol 2011; 108:1188-1195, 2011]. However, another study showed that the predictive quality of copeptin for major adverse cardiac events was lost when evaluating patients undergoing vascular surgery while in severe stages of chronic kidney disease (CKD) [Schrimpf et al., PLoS ONE 10(4):e0123093. DOI: 10.1371/journal.pone.0123093, 2015]. In summary, while there have been indications about possible predictive value of biomarkers for the outcome of postoperative patient's health, there is no clear method allowing an objective assessment of a patient's situation. There is therefore a need for methods allowing a quick and early assessment of possible MACCE and MINS in patients undergoing abdominal surgery, specifically gastrointestinal surgery, to reduce postoperative complications prolonging recovery and hospitalisation and increasing mortality.

A primary objective of the present invention therefore resides in the provision of a method and kit which allow a quick and reliable diagnosis, prognosis and rule-out of MACCE and/or MINS in patients who undergo major gastrointestinal surgery. Further objects will become apparent from the following description.

The objective is reached by a method and a kit according to the independent claims. Preferred embodiments are described in the respective dependent claims.

Specifically, the invention relates to a method for at least one of prognosis, risk assessment and diagnosis of a major adverse cardio- or cerebrovascular event (MACCE) in a patient who has undergone major gastrointestinal surgery, the method comprising the steps of:
i) providing a sample of a bodily fluid from said patient,
ii) determining in said sample the level of a biomarker selected from the group consisting of copeptin, troponin and brain natriuretic peptide (BNP),
iii) determining at least one additional parameter of said patient,
iv) combining the biomarker level determined in step ii) and the additional parameter determined in step iii) into a combined assessment, and
v) correlating the combined assessment to said at least one of prognosis, risk assessment and diagnosis of a MACCE in said patient.

Especially, the invention relates to a method for the diagnosis of a major adverse cardio- or cerebrovascular event (MACCE) in a patient who underwent gastrointestinal surgery, wherein step v) relates to correlating the combined assessment to the presence or absence of a MACCE in said patient.

The inventors realized that the specific biomarkers copeptin, troponin and BNP allow a quick and early diagnosis of whether or not a patient who underwent gastrointestinal surgery has MACCE. Moreover the inventors realized that the specific biomarkers were able to provide important prognostic information by identifying patients at risk of MACCE prior to surgery. The combination of the level of at least one of these biomarkers with at least one additional parameter shows improved results so that the resulting assessment outperforms those of each of the biomarkers alone. While the invention pertains to the field of clinical diagnostics, the determination of the biomarker levels is carried out on samples taken from the patient and is thus not practiced on the body of the patient.

The at least one additional parameter can be selected from the group consisting of the level of at least one additional biomarker and a clinical parameter of said patient. When using more than one additional parameter in the combined assessment, the additional parameters can be of the same category or different categories. In case of two additional parameters, for example, the additional parameters can both be a level of an additional biomarker, two clinical parameters or the level of an additional biomarker plus a clinical parameter. The additional biomarker level can be the level of one of the two remaining biomarkers mentioned in step ii) not used for determining the biomarker level in said step. If, for example, the biomarker level determined in step ii) is that of copeptin, the additional parameter may be the level of troponin or BNP.

More generally speaking, determining at least one additional parameter preferably comprises determining the level of at least one additional biomarker which is selected from the group consisting of copeptin, troponin, BNP, mid-regional proadrenomedullin (MR-proADM), C-terminal proendothelin-1 (CT-proET-1), procalcitonin (PCT), MR-proANP (mid-regional pro atrial natriuretic peptide), creatinine kinase, creatine kinase-MB, myoglobin, lactate and CRP (C-reactive protein).

In a preferred embodiment, the levels of all three biomarkers copeptin, troponin and brain natriuretic peptide (BNP) are determined and used in the following steps iv) and v). Optionally, at least one of MR-proADM, CT-proET-1 and PCT can additionally be determined and combined into the combined assessment.

The sequence of the 164 amino acid precursor peptide of Vasopressin (pre-pro-Vasopressin) is given in SEQ ID NO:1. Pro-Vasopressin relates to the amino acid residues 19 to 164 of the sequence of pre-pro-Vasopressin. The amino acid sequence of pro-Vasopressin is given in SEQ ID NO:2. Pro-Vasopressin is cleaved into mature Vasopressin, Neurophysin II and C-terminal proVasopressin (CT-proAVP or copeptin). Copeptin relates to amino acid residues 126 to 164 of pre-pro-Vasopressin. The amino acid sequence of copeptin is provided in SEQ ID NO:3. Neurophysin II comprises the amino acid residues 32 to 124 of pre-pro-Vasopressin and its sequence is shown in SEQ ID NO:4.

Troponin is a protein found in muscles which facilitate contraction by sliding of actin and my-osin filaments. It comprises three subunits, C, I and T. An isoform of troponin T, cTnT, is only found in cardiomyocytes and is the most important cardiac biomarker because of its high myocardial specificity and clinical sensitivity. However, troponin levels typically show a delay in their increase after surgery, so that their usefulness for early diagnosis of MACCE and MINS in post-operative patients has typically been regarded as limited. The sequence of the subunit C is given in SEQ ID NO:7. The sequence of the subunit I is given in SEQ ID NO:6. The sequence of the subunit T is given in SEQ ID NO:5.

The sequence of the 134 amino acid precursor peptide of brain natriuretic peptide (pre-pro-BNP) is given in SEQ ID NO:8. Pro-BNP relates to amino acid residues 27 to 134 of pre-pro-BNP. The sequence of pro-BNP is shown in SEQ ID NO:9. Pro-BNP is cleaved into N-terminal pro-BNP (NT-pro-BNP) and mature BNP. NT-pro-BNP comprises the amino acid residues 27 to 102 and its sequence is shown in SEQ ID NO:10. The SEQ ID NO:11 shows the sequence of BNP comprising the amino acid residues 103 to 134 of the pre-pro-BNP peptide.

A clinical parameter in the sense of this invention generally means any parameter indicative of the patient's health and/or physical condition. The clinical parameter is preferably selected from a parameter which is significant of a cardio- or cerebrovascular condition or event. Preferably, the clinical parameter is selected from the group consisting of age, abnormal ECG (especially pathological Q-waves), LBBB (development of left bundle branch block), ST-elevation (≥2 mm in V1, V2, V3 or ≥ 1mm in other leads), ST-depression (development of ST-segment depression ≥ 1mm), T-wave inversion (symmetric inversion of T-waves ≥ 1mm in at least two continuous leads), intraoperative hypotension, intraoperative tachycardia, intraoperative bradycardia, hyperlipidaemia, smoking status, anaemia, functional capacities (METs), red-blood cell transfusion, arrhythmias, rhythm other than sinus, duration of the gastrointestinal surgery and operation size, patient history (especially in regard of cardiac and cerebrovascular events) and renal disease. The invention also comprises any of the parameters as mentioned herein alone or in any combination with any number of the other parameters.

As there is no distinct, explicit and exclusive definition of MACCE and which conditions this term includes in the art, MACCE as used herein is defined as the occurrence of at least one condition selected from the following group of conditions:
- Non-fatal cardiac arrest (absence of cardiac rhythm or presence of chaotic rhythm requiring any component of basic or advanced cardiac life support),
- acute myocardial infarction (increase and gradual decrease in troponin level or a faster increase and decrease if creatine kinase isoenzyme as marker of myocardial necrosis in the company of at least one of the following: Ischemic symptoms, abnormal Q-waves on the ECG, ST-segment elevation or depression, coronary artery intervention (e.g. coronary angioplasty) or an atypical decrease in an elevated troponin level detected at its peak after surgery in a patient without documented alternative explanation for the troponin elevation),
- congestive heart failure (new in-hospital signs or symptoms of dyspnea or fatigue, orthopnea, paroxysmal nocturnal dyspnea, increased jugular venous pressure, pulmonary rales on physical examination, cardiomegaly or pulmonary vascular engorgement),
- new cardiac arrhythmia (ECG evidence of atrial flutter, atrial fibrillation, or second- or third-degree atrioventricular conduction block),
- angina (dull diffuse substernal chest discomfort precipitated by exertion or emotion and relieved by rest or glyceryl trinitrate),
- stroke (embolic, thrombotic, or hemorrhagic event lasting at least 30 min with or without persistent residual motor, sensory, or cognitive dysfunction; if the neurological symptoms continue for >24 h, a person is diagnosed with stroke, and if lasting <24 h the event is defined as a transient ischemic attack),
- transient ischemic attack (embolic, thrombotic, or hemorrhagic event lasting at least 30 min with or without persistent residual motor, sensory, or cognitive dysfunction; if the neurological symptoms continue for >24 h, a person is diagnosed with stroke, and if lasting <24 h the event is defined as a transient ischemic attack) and
- myocardial injury after noncardiac surgery (MINS; postoperative troponin elevation judged to be due to myocardial ischemia (no evidence of nonischemic etiology) with or without ischemic symptoms).
Other conditions not mentioned above, for example, circulatory shock, are therefore not considered a MACCE as used herein and are explicitly excluded.

"Diagnosis" in the context of the present invention relates to the recognition and early detection of a disease or clinical condition or its absence in a subject and may also comprise differential diagnosis and rule outs. Early in this regard is intended to mean the earliest possible moment for a diagnosis. This includes patients with and without disease related symptoms. The diagnosis is realized by the measurement of biomarkers with or without the addition of further clinical parameters, scores or medicinal procedures known in the art.

In the present invention, the term "risk assessment" denotes an assignment of a probability to experience certain adverse events to an individual. Hereby, the individual may preferably be accounted to a certain risk category, wherein categories comprise for instance high risk versus low risk, or risk categories based on numerical values, such as risk category 1, 2, 3, etc.

In the present invention, the term "prognosis" denotes a prediction of how a subject's (e.g. a patient's) medical condition will progress. This may include an estimation of the chance of recovery or the chance of an adverse outcome for said subject (e.g. occurrence of a MACCE or death).

The term "sample" as used herein refers to a sample of bodily fluid obtained for the purpose of diagnosis, prognosis, or evaluation of a subject of interest, such as a patient. Preferred test samples include blood, serum, plasma, cerebrospinal fluid, urine, saliva, sputum, and pleural effusions. In addition, one of skill in the art would realize that some test samples would be more readily analyzed following a fractionation or purification procedure, for example, separation of whole blood into serum or plasma components.

Thus, in a preferred embodiment of the invention the sample is selected from the group comprising a blood sample, a serum sample, a plasma sample, a cerebrospinal fluid sample, a saliva sample and a urine sample or an extract of any of the aforementioned samples. Preferably, the sample is a blood sample, most preferably a serum sample or a plasma sample.

The term "level" in the context of the present invention relates to the concentration (preferably expressed as weight/volume or molar amount/volume) of marker peptides taken from a sample of a patient.

The term "patient" as used herein refers to a living human or non-human organism that is receiving medical care or that should receive medical care due to a disease. This includes persons with no defined illness who are being investigated for signs of pathology, specifically persons who are investigated for a possible MACCE or MINS. Thus the methods and assays described herein are applicable to both, human and veterinary disease. Specifically, the patients evaluated in the inventive method undergo, preferably an elective, gastrointestinal surgery. The patient can also suffer from other comorbidities like hypertension, diabetes, COPD (chronic obstructive pulmonary disease), metabolic syndrome, obesity (BMI (body mass index) of at least 30), non-cardiovascular chronic disease, pain, fever, infection, sepsis or SIRS (systemic inflammatory response syndrome).

The term "combining" as used herein for the provision of a combined assessment, refers to the assignment of an indicative value to a measured or calculated result and the combination of values from different results into the combined assessment. It may comprise selection of a certain number of parameters and the arrangement of these parameters into specified groups using a mathematical algorithm (e.g. deviation or ratio). For example, a measured biomarker level can lie above or below a predetermined threshold, which the skilled person can translate into an indicative value by evaluating whether or not an increase in the biomarker value in comparison to reference data is considered indicative of the presence of a disease or condition. Different thresholds may provide different indicative strengths. The same applies to calculated results, e.g. decrease or increase ratios, ratios between different biomarker levels and so on. The term "combined assessment" therefore relates to the indicative value of the entirety of all measured or calculated results as provided by the present invention, which can then be correlated to a condition in a patient.

The term "correlating", as used herein in reference to the combined assessment, refers to comparing the entirety of the indicative value of all measured or calculated results (the combined assessment) to their value in persons known to suffer from, or known to be at risk of, a given condition. An assessment in a patient sample can be compared to a value known to be associated with a specific diagnosis. The sample's assessment is said to have been correlated with a diagnosis; that is, the skilled artisan can use the assessment to determine whether the patient suffers from a specific type of a disease, and respond accordingly. Alternatively, the assessment can be compared to an assessment known to be associated with a good outcome (e.g. the absence of disease etc.). In preferred embodiments, a panel of marker levels and their combined indicative value is correlated to a global probability or a particular outcome.

In the present invention, all of the patients the inventive method is practiced upon undergo a gastrointestinal surgery. The aim of the inventive method is to determine whether or not these patients have or are at risk of a MACCE. Therefore, the correlation of step iv) of the inventive method preferably includes providing reference data pertaining to patients who underwent gastrointestinal surgery and who did and/or did not have a MACCE or MINS. The combined assessment of the patient is then compared to this reference data to determine whether or not the patient has or is at risk of a MACCE or a MINS. A reference group consisting of patients who also underwent gastrointestinal surgery is better suited for the present invention than, for example, a reference group of healthy patients.

To increase the performance of the inventive method further, in a preferred embodiment, the level of at least one additional biomarker in the sample is determined, the additional biomarker being selected from the group consisting of copeptin, troponin, BNP, mid-regional proadrenomedullin (MR-proADM), C-terminal proendothelin-1 (CT-proET-1), procalcitonin (PCT), MR-proANP (mid-regional pro atrial natriuretic peptide), creatinine kinase, creatine kinase-MB, myoglobin, lactate and CRP (C-reactive protein). The level of the at least one additional biomarker is combined into the combined assessment. In an especially preferred embodiment, the levels of all of the biomarkers copeptin, troponin, BNP, MR-proADM, CT-proET-1 and PCT are determined and combined into the combined assessment. Employing a combination of biomarkers increases the diagnostic performance of the present method and makes the method less susceptible to fluctuations of the single biomarkers.

The sequence of the 153 amino acid pre-proANP is shown in SEQ ID NO:21. Upon cleavage of an N-terminal signal peptide (25 amino acids) and the two C-terminal amino acids, proANP (SEQ ID NO:22) is released. "Pro-atrial natriuretic peptide" or "proANP" refers to the pro-hormone comprising 126 amino acids. As used herein, a peptide comprising 28 amino acids (99-126) of the C-terminal section of a pro-hormone comprising 126 amino acids (proANP) is referred to as the actual hormone ANP. Upon release of ANP from its pro-hormone proANP, an equimolar amount of the remaining larger partial peptide of proANP, the N-terminal proANP, consisting of 98 amino acids (NT-proANP; proANP (1-98); shown as SEQ ID NO:23) is released into circulation. As NT-proANP possesses a significantly greater half life time and stability NT-proANP can be used as laboratory parameter for diagnosis, follow-up and therapy control; see, for example, Lothar Thomas (Editor), Labor und Diagnose, 5th expanded ed., sub-chapter 2.14 of chapter 2, Kardiale Diagnostik, pages 116-118, and WO 2008/135571. The level of proANP is preferably measured in the plasma or serum of a subject.

"Atrial natriuretic peptide (ANP)", a member of the natriuretic peptide family, regulates several physiological parameters including diuresis and natriuresis, and lower arterial blood pressure (BP). It is predominantly produced in the atrium of the heart and comprises 98% of natriuretic peptides in the circulation (Vesely DL. Life 2002;53:153-159). ANP is derived from the cleavage of its precursor pro-hormone, which is significantly more stable in the circulation than the mature peptide. A midregional fragment of the precursor hormone (amino acids 53-90 of NT-proANP; SEQ ID NO:24), called mid-regional-proANP (MR-proANP), may be relatively resistant to degradation by exoproteases, unlike epitopes in the N- or C-terminals of proANP used in previous immunoassays (Morgenthaler NG et al. Clin Chem 2004;50:234-236; Gerszten RE et al. 2008. Am J Physiol Lung Cell Mol Physiol). We have previously noted that in adults with essential hypertension, higher plasma levels of MR-proANP were associated with higher systolic blood pressure (SBP), greater arterial stiffness and severity of hypertension (unpublished data). Based on these findings, we hypothesized that plasma MR-proANP may be associated with measures of target organ damage in adults with hypertension.

As used herein, "lactate" refers to the lactate concentration measured in the blood. Normally, the lactate concentration is assessed daily or even more often. The lactate concentration in the blood can be determined by lactate oxidase spectrophotometric methods. As for clinical applications, it is well known in the art that lactate can identify patients with an increased morbidity and mortality risk (Broder G, Weil MH. Excess lactate: An index of reversibility of shock in human patients. Science 1964; 143: 1457-59; Manikis P, Jankowski S, Zhang H, Kahn RJ, VincentJL. Correlation of serial blood lactate levels to organ failure and mortality after trauma. Am J Emerg Med 1995; 13,6: 619-22.)

"Myoglobin" is a single-chain globular protein of 153 amino acids (SEQ ID NO:26), containing a heme (iron-containing porphyrin) prosthetic group in the center around which the remaining apoprotein folds. Its sequence is shown in SEQ ID NO:25. Myoglobin is a sensitive marker for muscle injury, making it a potential marker for heart attack in patients with chest pain (Weber et al. 2005. Clinical Biochemistry 38: 1027-30). CK-MB and cardial troponin T cTnT are used in combination with ECG, and the clinical signs to diagnose Acute Myocardial Infarction (AMI). Cardial troponin is a protein complex consisting of the three subunits T (cTcT), I (cTcI) and C, of which T and I are only located in heat muscle tissue and are used as markers for diagnostic purposes (Rottbauer W et al., Eur Heart J 1996;17:3-8).

"Creatine kinase" is an enzyme and is represented by different isoforms that could have a B-(brain type) or M- (muscle type) subunit. Creatine kinase comprises the isoforms CK-MM, CK-MB and CK-BB, that can have distinct expression pattern based on the tissue type (Schlattner U, Tokarska-Schlattner M, Wallimann T (February 2006). "Mitochondrial creatine kinase in human health and disease". Biochimica et Biophysica Acta. 1762 (2): 164-80.)The enzyme plays a functional role in the adenosine triphosphate (ATP) metabolism and is therefore present in organs such as muscle, retina, brain, heart or kidney that have a high demand for ATP. Blood levels of creatine kinase are detected to detect tissue damage in associated diseases as for example myocardial infarction, muscular dystrophy or acute kidney injury. (Wallimann T, Wyss M, Brdiczka D, Nicolay K, Eppenberger HM (January 1992). "Intracellular compartmentation, structure and function of creatine kinase isoenzymes in tissues with high and fluctuating energy demands: the 'phosphocreatine circuit' for cellular energy homeostasis". The Biochemical Journal. 281 (Pt 1) (1): 21-40; Moghadam-Kia S, Oddis CV, Aggarwal R (January 2016). "Approach to asymptomatic creatine kinase elevation". Cleveland Clinic Journal of Medicine. 83 (1): 37-42).

"C-reactive protein (CRP)" is an acute-phase protein, which is physiologically induced during inflammatory conditions (Thompson D, Pepys MB, Wood SP (1999). "The physiological structure of human C-reactive protein and its complex with phosphocholine". Structure. 7 (2): 169-77). The sequence of CRP is given in SEQ ID NO:27. CRP is part of the complement system by promoting phagocytosis of Macrophages. Moreover, CRP can also activate the innate immune response against infections (Bray C, Bell LN, Liang H, Haykal R, Kaiksow F, Mazza JJ, Yale SH (December 2016). "Erythrocyte Sedimentation Rate and C-reactive Protein Measurements and Their Relevance in Clinical Medicine"). Wisconsin Medical Journal (WMJ). 115 (6): 317-21.). CRP is known and widely used as a marker for inflammation. In healthy adults, the normal concentrations of CRP varies between 0.8 mg/L to 3.0 mg/L and in rare occasions up to 10 mg/L. Pathological levels can be defined as follows: mild inflammation and viral infections (10-40 mg/L), active inflammation, bacterial infection (40-200 mg/L), severe bacterial infections and burns (>200 mg/L) (Chew KS (April 2012). "What's new in Emergencies Trauma and Shock? C-reactive protein as a potential clinical biomarker for influenza infection: More questions than answers". Journal of Emergencies, Trauma, and Shock. 5 (2): 115-7.).

The amino acid sequence of the precursor peptide of Adrenomedullin (pre-pro-Adrenomedullin) is given in SEQ ID NO:12. Pro-Adrenomedullin relates to amino acid residues 22 to 185 of the sequence of pre-pro-Adrenomedullin. The amino acid sequence of pro-Adrenomedullin (proADM) is given in SEQ ID NO:13. MR-pro-Adrenomedullin (MR-proADM) relates to amino acid residues 45-92 of pre-proADM. The amino acid sequence of MR-proADM is provided in SEQ ID NO:14.

The sequence of the 212 amino acid precursor peptide of Endothelin-1 (pre-pro-Endothelin-1) is given in SEQ ID NO:15. Pro-ET-1 relates to the amino acid residues 18 to 212 of the sequence of pre-pro-ET-1. The amino acid sequence of pro-ET-1 is given in SEQ ID NO:16. Pro-ET-1 is cleaved into mature ET-1, big-ET-1 and C-terminal proET-1 (CT-proET-1). ET-1 relates to the amino acid residues 53 to 73 of pre-pro-ET-1. The amino acid sequence of ET-1 is shown in SEQ ID NO:17. CT-proET-1 relates to amino acid residues 168 to 212 of pre-pro-ET-1. The amino acid sequence of CT-proET-1 is provided in SEQ ID NO:18. Big-ET-1 comprises the amino acid residues 53 to 90 of pre-pro-ET-1 and its sequence is shown in SEQ ID NO:19.

Procalcitonin is a precursor of calcitonin and katacalcin. The amino acid sequence of PCT 1-116 is given in SEQ ID NO:20.

Generally, the levels of the mentioned biomarkers can be measured by directly determining the biomarkers themselves. However, in a preferred embodiment, the level of at least one of the biomarkers is determined by determining the level of at least one of a precursor, a precursor fragment and a fragment of the biomarker. In other words, the levels of the biomarkers are determined indirectly by determining the levels of precursors, precursor fragments or fragments of the biomarkers. The advantage lies in the fact that these indirect peptide targets are often more easily measured or are more stable either in the patient's bodily fluid or in the assay environment. As the ratios of the indirect peptide targets to the biomarkers of interest are known, the levels of the indirect peptide targets can easily be used to determine the levels of the biomarkers of interest.

As mentioned herein in the context of proteins and other peptides, the term "fragment" refers to smaller proteins or peptides derivable from larger proteins or peptides, which hence comprise a partial sequence of the larger protein or peptide. Said fragments are derivable from the larger proteins or peptides by saponification of one or more of its peptide bonds. "Fragments" of the biomarkers preferably relate to fragments of at least 6 amino acids in length, most preferably at least 12 amino acid residues in length. Such fragments are preferably detectable with immunological assays.

For example, in a preferred embodiment, determining the level of troponin comprises determining the level of the subunit cardiac troponin T (cTnT), preferably isoform 6 of cTnT (SEQ ID NO:5) or a homologous peptide with at least 75% amino acid sequence identity with isoform 6 of cTnT. More preferably, the amino acid sequence identity with isoform 6 of cTnT is at least 80%, at least 85%, at least 90% and, most preferably, at least 95%. Cardiac troponin T is released from the myocardium through tissue damage and is therefore used as a biomarker for MI. There are several isoforms of cTnT and the current invention is not restricted to either one of them, while isoform 6 is preferred. However, any of the other isoforms can also be used in the present invention.

In another preferred embodiment, determining the level of BNP comprises determining the level of precursor fragment NT-proBNP (SEQ ID NO:10).

As the risk for postoperative complications increases with patient age, it is preferred that the inventive method is practiced on patients who are at least 50 years old. Limiting the patient population on which the method is practiced enables cut-off values to be specifically chosen for the biomarker levels prevalent in the target population. By evaluating patients with at least 50 years of age, the inventive method can be tailored to the physiological parameters of this group.

The present invention is directed to the diagnoses of serious complications following gastrointestinal surgery. Therefore, as severe complications are more predominant after severe surgical procedures, it is preferred that the patient spent at least one day in a hospital after undergoing the gastrointestinal surgery. The length of the hospital stay reflects the severity of the surgical procedure and therefore the general risk of complications. The invention can also be practiced on a patient being treated with analgesics or pain treatment. By directing the method to this group of patients, the inventive diagnosis is directed to the ones who potentially are most in need.

Eligible gastrointestinal surgery procedures can be either laparoscopic or open (conventional). Specifically, the gastrointestinal surgery is selected from the group consisting of
- stomach surgery,
- small intestine surgery, in particular duodenum surgery,
- large intestine surgery, in particular rectum surgery,
- reproductive system surgery, in particular hysterectomy or salpingoophorectomy,
- kidney surgery, in particular nephrectomy,
- urinary bladder surgery, in particular cystectomy,
- gallbladder surgery, in particular cholecystectomy, and
- surgery of gastrointestinal cysts.

As the present method is directed to the diagnosis of MACCE resulting from gastrointestinal surgery, it is preferred that the patient is free from cardiovascular comorbidities. Such comorbidities often also lead to nonphysiological levels of the biomarkers as described herein and could thus obscure or be mistaken as newly acquired MACCE as a result of the gastrointestinal surgery. The diagnostic value of the present method is therefore higher in patients without pre-existing conditions that could be mistaken as MACCE.

Accordingly, in a preferred embodiment, the patient is excluded if an exclusion condition is present in the patient, the exclusion condition being selected from the group consisting of organ transplantation, traumatic injury, endocrine disease, endocrine surgery, vascular disease, vascular surgery (e.g. surgery of an aneurism), endovascular disease, endovascular surgery, acute coronary syndrome (ACS), heart failure, decompensated congestive heart failure, aortic stenosis, reduced left ventricular ejection fraction (LVEF), and circulatory shock. The exclusion condition can either lie or have been diagnosed in the recent past of the patient or be diagnosed shortly after the gastrointestinal surgery. The main criterion is that the exclusion condition present in the patient influences the levels of the biomarkers according to the present method at the time of sampling of the patient. If this is the case and these circumstances are timely identified, the patient is excluded. In this way, the detrimental effect of the exclusion condition on the diagnostic performance of the present invention can be prevented. Otherwise, the present method might lead to the wrong diagnosis of a MACCE in a patient under the influence of one or more of the exclusion conditions.

As the present invention aims at a quick diagnosis of a MACCE in the aftermath of gastrointestinal surgery, the sample is preferably taken at a time shortly before and/or after the gastrointestinal surgery or within the first three days after the surgery. Specifically, it is preferred that the sample has been taken from the patient no more than 24 hours before the gastrointestinal surgery, no more than 24 hours after the gastrointestinal surgery, on day one after the gastrointestinal surgery, on day two after the gastrointestinal surgery or on day three after the gastrointestinal surgery. While taking the sample after the gastrointestinal surgery is of course suitable to monitor the effects of the gastrointestinal surgery itself on the level of the biomarkers, taking the sample shortly before the gastrointestinal surgery has two main applications. First of all, it serves to establish a baseline of the levels of the biomarkers as described herein in the specific patient. The levels of the biomarkers taken at later points in time therefore can be compared to these baselines. Second, higher levels of the biomarkers as mentioned herein in comparison to a healthy control group even in patients without one of the previously mentioned exclusion conditions can signify an increased risk of this specific patient for developing a MACCE after the gastrointestinal surgery. Therefore, the measurement of the levels of the biomarkers prior to the gastrointestinal surgery can serve to increase the diagnostic value of the inventive method. The control group can also be a group of patients who underwent gastrointestinal surgery and did or preferably did not develop a MACCE or MINS.

The inventive method yields valuable results even when only one sample of the patient taken at one of the previously mentioned points in time is evaluated. However, the performance of the inventive method is increased when multiple samples of the patient taken at different points in time are evaluated. In a preferred embodiment, the inventive method therefore comprises providing of from two to five samples from the patient, wherein the samples are taken at different times before and/or after the gastrointestinal surgery, and wherein steps ii), iv) and v) are practiced on all said samples. In the most preferred embodiment, five samples are taken from the patient at different points in time shortly before and after the gastrointestinal surgery. Preferably, the different times said of from two to five samples are taken are selected from the group consisting of no more than 24 hours before the gastrointestinal surgery, no more than 24 hours after the gastrointestinal surgery, day one after the gastrointestinal surgery, day two after the gastrointestinal surgery and day three after the gastrointestinal surgery. In the embodiment wherein five samples are taken, the respective sample is taken at one of these points in time. The determination of the biomarker levels in these samples can either be done sequentially, for example each sample can be measured on the day it is taken. Alternatively, the samples from all points of time can be collected and measured simultaneously, for example after the last sample has been taken. For different biomarkers, different assays or one multi-marker collective assay can be used.

In the embodiments of the present method in which more than one sample of the patient is provided, the development of the levels of the biomarkers in the samples, for example an increase or decrease in the biomarker levels between the points in time the samples were taken, can be used to increase the diagnostic value of the invention. For example, in a preferred embodiment, the levels of said biomarkers in the samples taken at different times are determined and then compared, and the differences in the levels of the biomarkers at different times are combined into the combined assessment. By comparison of the levels in samples taken at different times, additional information about the condition of the patient can be obtained, going beyond the biomarker levels at a specific time. As already mentioned, this additional information can be an increase or a decrease of a specific biomarker over the period in which samples were taken. However, additional information can also be gained through a more complex evaluation of the relationship of each of the biomarkers to the others. For example, the significance of a high level of copeptin on day one after the gastrointestinal surgery, generally being an indicator for a MACCE, is either increased if the patient also shows a high level of troponin and/or BNP on day two and/or three or decreased if the level of troponin and/or BNP does not show an increase at these times. In this way, the levels of the biomarkers measured in the samples taken at different times develop a synergy in the diagnostic value of the inventive method, going way beyond the mere levels of the biomarkers at one or more points in time (i.e. the information that a biomarker level dropped by a certain value from one sampling time to another has more diagnostic value than the information about the biomarker levels at these sampling times alone).

In a preferred embodiment, a biomarker level determined to be above a specific threshold value is indicative of a MACCE in the patient. For the value of this specific threshold, several factors have to be taken into account, as will be explained below.

First of all, the sensitivity and specificity of a diagnostic and/or prognostic test depend on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy; herein specifically patients undergoing gastrointestinal surgery and not developing a MACCE) and "disease" populations (i.e. patients suffering from diabetes, insulin resistance and/or metabolic syndrome; herein specifically patients undergoing gastrointestinal surgery and developing a MACCE). For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve (AUC) is a measure of the probability that the measured marker level will allow correct identification of a disease or condition. Thus, the area under the ROC curve can be used to determine the effectiveness of the test. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art (See, *e.g.,* Hartley et al. 1982. Radiology 143: 29-36). Preferably, ROC curves result in an AUC of greater than about 0.5, more preferably greater than about 0.7, still more preferably greater than about 0.8, even more preferably greater than about 0.85, and most preferably greater than about 0.9. The term "about" in this context refers to +/- 5% of a given measurement. In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

It is therefore important to note that the specific threshold values used in the inventive method have to be adapted to the use of the invention in practice. Different threshold values will lead to different sensitivity and specificity of the invention, which should be adapted by the clinician using the invention with consideration of the specific situation. Thus, one might adopt the threshold value depending on whether it is considered more appropriate to identify most of the subjects at risk at the expense of also identifying "false positives", or whether it is considered more appropriate to identify mainly the subjects at high risk at the expense of missing several subjects at moderate risk. These decisions have to be taken by the clinician practicing the present invention and are implemented by a modification of the threshold values. The threshold values as given herein are therefore exemplary and are not to be understood to restrict the invention in any way.

Another point to consider is that different assays may need different threshold values if these assays have been calibrated differently. Therefore the above mentioned threshold values shall apply for such differently calibrated assays accordingly, taking into account the differences in calibration. One possibility of quantifying the difference in calibration is a method comparison analysis (correlation) of the assay in question (e.g. a copeptin assay) with the respective biomarker assay used in the present invention (e.g. Thermo Scientific B·R·A·H·M·S Kryptor Compact plus) by measuring the respective biomarker (e.g. copeptin) in samples using both methods. Another possibility is to determine with the assay in question, given this test has sufficient analytical sensitivity, the median biomarker level of a representative normal population, compare results with the median biomarker levels as described in the literature and recalculate the calibration based on the difference obtained by this comparison.

All in all, the specific threshold values used in the present invention have to be determined on an individual basis whenever the invention is employed. As exemplary values, and in a preferred embodiment of the invention, the specific threshold value above which a biomarker level is indicative of a MACCE in the patient is selected from the group consisting of
- 25 pmol/L, preferably 75 pmol/L, more preferably 125 pmol/L, even more preferably 175 pmol/L and most preferably 225 pmol/L for copeptin,
- 15 ng/L, preferably 20 ng/L, more preferably 25 ng/L, even more preferably 30 ng/L and most preferably 35 ng/L, 45 ng/L or 65 ng/L for cTnT,
- 500 ng/L, preferably 900 ng/L, more preferably 1300 ng/L, even more preferably 1700 ng/L and most preferably 2100 ng/L or 2800 ng/L for NT-proBNP,
- 0.8 nmol/L or 1.0 nmol/L, preferably 1.25 nmol/L, more preferably 1.5 nmol/L, even more preferably 1.75 nmol/L and most preferably 2.0 nmol/L or 2.4 nmol/L for MR-proADM,
- 80 pmol/L, preferably 90 pmol/L, more preferably 100 pmol/L, even more preferably 110 pmol/L and most preferably 120 pmol/L for CT-proET-1, and
- 0.5 *µ*g/L, preferably 1.0 *µ*g/L, more preferably 1.5 *µ*g/L, even more preferably 2.0 *µ*g/L and most preferably 2.5 *µ*g/L or 3.0 *µ*g/L for PCT.
In another specific embodiment, the threshold lies within a margin of plus or minus 10% of any of the above-mentioned thresholds. The threshold for copeptin could thus for example also be anywhere between 22.5 pmol/L and 27.5 pmol/L (e.g. in a range of 25 pmol/L plus or minus 10%). This applies to all the exemplary threshold values given herein.

In further specific embodiments, the risk of a MACCE in the patient pertains to the risk of a MACCE in the patient within at least one of 30 days and 12 months after the gastrointestinal surgery. These time spans have proven to be most important in clinical prognosis of MACCE.

Apart from the mere occurrence of a MACCE, the present invention may also be used to assess the risk of death of the patient, specifically death from developing a MACCE after gastrointestinal surgery. It is therefore preferred that the prognosis comprises the risk of mortality within at least one of 30 days and 12 months after the gastrointestinal surgery.

The important subgroup of MACCE, myocardial injury after noncardiac surgery (MINS) has already been mentioned. As MINS is responsible for a large number of complications and prolonged hospitalizations including increased patient mortality, in a preferred embodiment of the invention, the MACCE is a MINS and the method is a method for the diagnosis of a MINS in a patient who underwent gastrointestinal surgery. In other words, the inventive method can be directed to the specific diagnoses and/or prognosis pertaining to MINS in a patient who underwent gastrointestinal surgery. As MINS typically do not sufficiently show any specific symptoms in other clinical tests, including ECG, the invention is of high practical value as patients developing a MINS might otherwise go undetected and therefore untreated.

In a preferred embodiment of the invention, therefore, a biomarker level determined to be above a specific threshold value is indicative of a MINS in the patient. The general remarks pertaining to the definition and the determination of specific threshold values as mentioned above of course also apply to this specific threshold values in connection with MINS.

In another preferred embodiment of the invention, a biomarker level determined to be above a specific threshold value is indicative of a perioperative myocardial injury in the patient. The general remarks pertaining to the definition and the determination of specific threshold values as mentioned above of course also apply to this specific threshold values in connection with perioperative myocardial injury.

As exemplary values, and in a preferred embodiment of the invention, the specific threshold value above which a biomarker level is indicative of a MINS in the patient is selected from the group consisting of
- 25 pmol/L, preferably 50 pmol/L or 75 pmol/L, more preferably 125 pmol/L, even more preferably 175 pmol/L and most preferably 225 pmol/L for copeptin,
- 10 ng/L, preferably 20 ng/L, more preferably 30 ng/L, even more preferably 40 ng/L and most preferably 50 ng/L or 60 ng/L for cTnT,
- 250 ng/L or 500 ng/L, preferably 750 ng/L, more preferably 1250 ng/L or 1500 ng/L, even more preferably 1750 ng/L and most preferably 2250 ng/L for NT-proBNP,
- 0.8 nmol/L or 1.0 nmol/L, preferably 1.25 nmol/L, more preferably 1.5 nmol/L, even more preferably 1.75 nmol/L and most preferably 2.0 nmol/L for MR-proADM,
- 65 pmol/L or 75 pmol/L or 80 pmol/L, preferably 90 pmol/L, more preferably 100 pmol/L, even more preferably 110 pmol/L and most preferably 120 pmol/L for CT-proET-1, and
- 0.2 *µ*g/L or 0.5 *µ*g/L, preferably 0.75 *µ*g/L or 0.8 *µ*g/L, more preferably 1.0 *µ*g/L, even more preferably 1.25 *µ*g/L and most preferably 1.5 *µ*g/L for PCT.
In another specific embodiment, the threshold lies within a margin of plus or minus 10% of any of the above-mentioned thresholds. The threshold for copeptin could thus for example also be anywhere between 22.5 pmol/L and 27.5 pmol/L (e.g. in a range of 25 pmol/L plus or minus 10%). This applies to all the exemplary threshold values given herein.

One further option when determining the specific threshold values above which the level of the biomarker is indicated of a MACCE and/or MINS is to also considering the point in time when the sample in question was taken from the patient. For example, the level of a specific biomarker, for example, copeptin, is elevated shortly after the gastrointestinal surgery both in patients developing a MACCE and/or MINS and in patients who do not develop such complications. This increase, however, quickly drops again. Therefore, when evaluating the levels of copeptin, a higher specific threshold should be chosen for the sample taken at most 24 hours after the gastrointestinal surgery in comparison to any other point in time. This can be done for any of the biomarkers as mentioned herein. It is therefore a preferred embodiment of the present invention, that the determined biomarker levels taken at different times are evaluated using different threshold values. In this way, the physiological baseline of the biomarker levels as determined in patients not developing a MACCE and/or MINS can be considered and taken into account in the inventive method.

Another increase in the diagnostic performance of the invention may be achieved if the determined biomarker levels taken at different times are differently weighted. For example, an increased level of troponin in samples taken on day two and/or on day three after the gastrointestinal surgery is more significant than an increased level of troponin in a sample taken before the gastrointestinal surgery. Therefore, increased levels on day two and/or on day three after the gastrointestinal surgery can be given more diagnostic weight in the combined assessment, meaning that these increased levels at these specific points in time influence the combined assessment more than less significant measurements at other points in time. The different weight assigned to biomarker levels taken at different times can also pertain to different biomarkers. For example, an increased level of troponin in samples taken on day two and/or on day three after the gastrointestinal surgery can be more significant than an increased level of copeptin on day one and/or two and/or three after the gastrointestinal surgery. By weighting the determined biomarker levels according to their diagnostic value at the specific points in time of their respective measurement, the performance of the invention can be increased.

By providing multiple samples from different points in time from the patient, these samples and their significance for the diagnosis and/or prognosis of the patient can be assessed in many different ways. For example, a relative change in the biomarker level between two samples taken at different times can be combined into the combined assessment. A relative change is measured as a decrease or an increase of the level of a specific biomarker expressed in relation to a previous level, for example, as a percentage. It can, for example, be determined by how much the biomarker increased or decreased in view to one or more previous measurements. These measurements do not have to be sequential. For example, the increase or decrease of the level of a biomarker could be determined from the measurement prior of the gastrointestinal surgery to the first and/or the second and/or the third and/or the fourth postoperative measurement. In this way, each of the samples taken at a specific point in time can be compared to any other sample from any other point in time. This can differ for each of the biomarkers. For example, the method could comprise using the relative change of BNP from a sample taken on the second day to the third day after the surgery and additionally the relative change of copeptin from the sample taken at most 24 hours after the gastrointestinal surgery to the sample taken on the first day after the surgery. Any such combinations are possible.

In another preferred embodiment of the present invention, a ratio between the levels of at least two biomarkers in the sample is determined and said ratio is combined into the combined assessment, wherein the biomarkers for determining the ratio are preferably selected from the pairs copeptin/troponin, copeptin/BNP and BNP/troponin. The level of the at least two biomarkers can be from one sample or of different samples, specifically different samples taken at different points in time. The ratio can be calculated between the measured levels of the biomarkers directly or parameters derived from the levels of the biomarkers, for example the relative change of the biomarker levels. In a preferred embodiment, the ratio between the relative changes of at least two biomarkers is determined and combined into the combined assessment. In another preferred embodiment, the ratios determined from samples taken at different times are compared to obtain a relative change of the ratios over time, preferably expressed as percentage, which is then also combined into the combined assessment.

For example, the method also comprises providing reference data for the determined biomarkers and especially for the determined parameters as described above, for example the difference between the biomarker levels at two different points in time, the relative change of the biomarker levels or the ratios of the biomarker levels. The reference data can then be used to compare the measured results and determine the status of the patient. In this preferred embodiment, step iv) comprises providing reference data for the determined biomarkers and at least one value selected from the group consisting of
- the determined level of at least one of the biomarkers,
- the differences in the level of at least one of the biomarkers taken at different times,
- the relative change in the levels of one of said biomarkers determined in two samples taken at different times,
- the ratio between the levels of at least two biomarkers either taken at the same time or taken at different times and
- the relative change of the ratios of at least two biomarkers determined in at least two samples taken at different times
is compared to the reference data, wherein the difference of the value as determined and the reference data is computed, and wherein the computed difference is preferably expressed in the form of a score, especially as a numerical value.

Reference data is preferably also provided with regard to a clinical parameter as the additional parameter. The reference data is again preferably provided from a reference group consisting of patients who also underwent gastrointestinal surgery, but reference data from a reference group of healthy patients is principally also suitable. As previously stated, the clinical parameter determined in the patient is compared to the reference data and the result is preferably transferred into a score, particularly a numerical value. While a clinical parameter can be determined at the same time(s) as the biomarker level(s) it will only make sense to take more than one value in case significant changes of the clinical parameter can be expected in the time frame in question. Clinical parameters, such as age, smoking habits, patient history and the like, need obviously only be taken once. Other clinical parameters, such as ECG, may be taken at several points in time. The relative change of the values thus obtained may be evaluated in an analogous way as described for the biomarker levels.

The term "score" in the context of the present invention refers to a rating, especially expressed numerically, based on the specific achievement or the degree to which certain qualities or conditions (e.g. the level of biomarkers or derived parameters) are present in said patient.

It is further preferred that at least two different scores are determined and the combined assessment comprises a combined score determined from said at least two different scores indicative of the presence or absence of a MACCE in the patient. In other words, the method comprises a scoring system in which each of the determined parameters, i.e., at least one biomarker level and at least one additional parameter, are expressed as scores, representing intermediate results, which are then combined into a combined score, from which the desired diagnosis and/or prognosis can be derived. For example, the reference data is divided into groups of values, with their values increasing or decreasing, preferably linearly. The increase or decrease is suitably in line with the severity of the outcome of the patients from which the reference data was taken. Each of the groups is associated with a respective score, for example, such that the number increases with severity. The values determined for the patient in accordance with steps ii) and iii) are then compared with the reference values associated with the predefined groups. The score of the appropriate group is then attributed to the determined parameter and used for evaluating the combined score, which then forms the basis for the final assessment. This evaluation is done for each of the parameters determined in steps ii) and iii), that is, the level of at least one biomarker and the at least one additional parameter. In the same way, biomarker levels at specific points in time or their differences or their relative changes or their ratios as described above can be evaluated and expressed as scores which are then used in the combined score.

As these scores and the combined score can be expressed as numerical values, they can also be graded according to the degree of their difference to the reference data. This results in a graded combined score, which may serve to stratify the patient into a specific risk group pertaining to the diagnosis and/or the prognosis of the patient having or developing a MACCE or MINS. Such risk groups may for example pertain to a low risk, a medium risk or a high risk of having or developing a MACCE or MINS, specifically in the time frames as mentioned above. The stratification can also pertain to the risk of death, also specifically in the time frames as mentioned above. The score as described can also be used for therapeutic guidance, with different risk groups being indicative of the need of different therapeutic approaches for the specific patient.

The reference data can be data obtained from healthy subjects. These subjects can be free from any known pathology and not undergo gastrointestinal surgery. However, it is preferred that the reference data pertains to patients who underwent gastrointestinal surgery and who did and/or did not have a MACCE or MINS. The comparison of the data obtained from the patient as described above with reference data from patients also undergoing gastrointestinal surgery but not developing a MACCE or MINS is especially meaningful, which is why this is the most preferred embodiment.

The invention also pertains to a kit for carrying out the method as explained herein. All of the features, effects and advantages of the inventive method also apply to the inventive kit and vice versa. To avoid repetitions, reference is therefore made to the previous explanations. Specifically, the inventive kit comprises at least one detection reagent for determining the level of at least one of the biomarkers copeptin, troponin and brain natriuretic peptide (BNP), in a sample of a bodily fluid from a patient, and reference data from patients who underwent gastrointestinal surgery, particularly reference levels, corresponding to at least one of copeptin, troponin and BNP levels, wherein said reference data is preferably stored on a computer-readable medium and/or employed in the form of computer-executable code configured for comparing the determined level of at least one of copeptin, troponin and BNP to said reference data. Especially preferably, the detection reagents comprise reagents for determining the levels of the biomarkers copeptin, troponin and brain natriuretic peptide (BNP) in a sample of a bodily fluid from said patient, and wherein the reference data comprises data corresponding to said biomarkers in patients who underwent gastrointestinal surgery.

The detection reagents can for example comprise capture molecules. They can, for example, be designed for use in an assay system such as the Thermo Scientific B·R·A·H·M·S Kryptor Compact plus. In a preferred embodiment of the kit, the detection reagents comprise also reagents for determining the level of at least one of the biomarkers mid-regional proadrenomedullin (MR-proADM), C-terminal proendothelin-1 (CT-proET-1) and procalcitonin (PCT) in a sample of a bodily fluid from said patient, and wherein the reference data comprise data corresponding to said at least one biomarker in patients who underwent gastrointestinal surgery.

Herein, the term "capture molecules" comprises molecules which may be used to bind target molecules or molecules of interest, i.e. analytes (e.g. the biomarkers), from a sample. Capture molecules must thus be shaped adequately, both spatially and in terms of surface features, such as surface charge, hydrophobicity, hydrophilicity, presence or absence of Lewis donors and/or acceptors, to specifically bind the target molecules or molecules of interest. Hereby, the binding may, for instance, be mediated by ionic, van-der- Waals, pi-pi, sigma-pi, hydrophobic or hydrogen bond interactions or a combination of two or more of the aforementioned interactions or covalent interactions between the capture molecules and the target molecules or molecules of interest. In the context of the present invention, capture molecules may for instance be selected from the group consisting of a nucleic acid molecule, a carbohydrate molecule, a PNA molecule, a protein, a peptide and a glycoprotein. Capture molecules include, for example, antibodies, aptamers, DARpins (Designed Ankyrin Repeat Proteins). Affimers and the like are included.

The term "antibody" as used herein, unless indicated otherwise, is used broadly to refer to both, antibody molecules such as monoclonal antibodies, polyclonal antiserum, enriched or purified polyclonal antibodies, recombinant antibodies and a variety of antibody-derived molecules, specifically functional derivatives. Such antibody-derived molecules comprise at least one variable region (either a heavy chain or a light chain variable region), as well as individual antibody light chains, individual antibody heavy chains, chimeric fusions between antibody chains and other molecules, and the like. Functional immunoglobulin fragments according to the present invention may be Fv, scFv, disulfide-linked Fv, Fab, and F(ab')2. Also encompassed by the term "antibody" are polyclonal antibodies, monoclonal antibodies, preferably IgG1 antibodies; chimeric monoclonal antibodies; humanized antibodies, genetically engineered monoclonal antibodies. Functional derivatives are chemically and/or bio-chemically modified variants of the antibodies/antisera having an analogous functionality/binding capacity.

The reference data as provided in the inventive kit is preferably designed to differentiate a patient between at least one of the pairs of having/not having and developing/not developing a MACCE or MINS after gastrointestinal surgery. The reference data is therefore preferably designed to be compared to the biomarker levels determined in samples taken from the patient at specific points in time as described above. Through the use of the inventive kit, a clinician may reach a quick and easy diagnosis and/or prognosis of a patient undergoing gastrointestinal surgery in view of the risk of having and/or developing a MACCE or MINS.

The invention also encompasses a computer running computer-executable code, with the computer-executable code being configured to carry out steps iv) and/or v) of the inventive method. For example, the computer-executable code is designed to create the combined assessment from input data, for example, the determined biomarker levels. In a preferred embodiment, the computer running the code is also connected to an assay system, the assay system being configured to carry out step ii) and, optionally, step iii) of the inventive method. The assay system can be, for example, a Thermo Scientific B·R·A·H·M·S Kryptor Compact plus. In this way, the assay system can provide the computer with the input data for carrying out steps iv) and/or v). The computer can also be provided with the aforementioned reference data. Optimally, a user merely has to carry out step i), while the computer with the assay system carry out the rest of the method and present the user with the resulting diagnosis and/or prognosis, or at least the score(s) on the basis of which the diagnosis and/or prognosis can be made.

Finally, the invention also relates to a computer program product comprising a set of computer instructions stored on at least one computer-readable medium, wherein said set of computer instructions further comprises instructions executable by one or more processors to carry out steps iv) and/or v) of the method of the invention. The computer-readable medium may be any kind of computer storage means, such as a USB stick, an external or internal hard disk, a diskette, a storage tape, an external handheld device, such as a mobile phone, tablet, or the like.

### BRIEF DESCRIPTION OF THE DRAWINGS

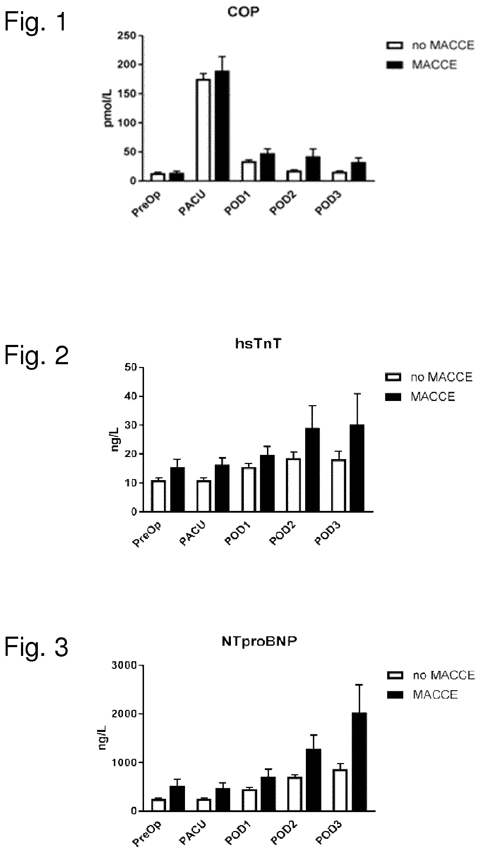

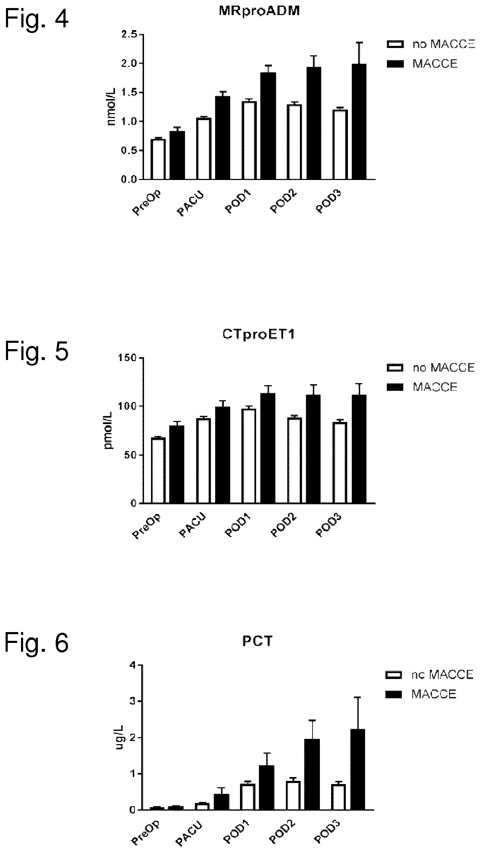

In the drawings, figures 1-6 show the determined levels of the biomarkers copeptin, cTnT (marked hsTnT for a high-sensitivity assay used, see below), NT-proBNP, MR-proADM, CT-proET-1 and PCT in a patient population at different times of sampling and divided in patients who did and did not develop a MACCE.

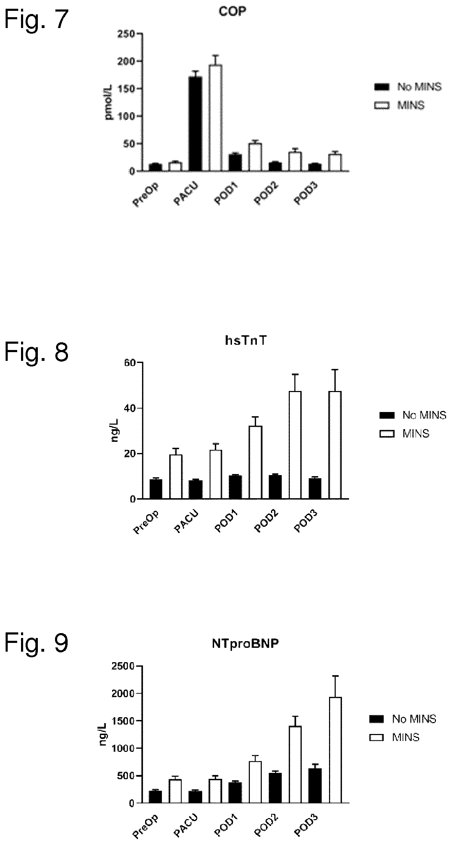

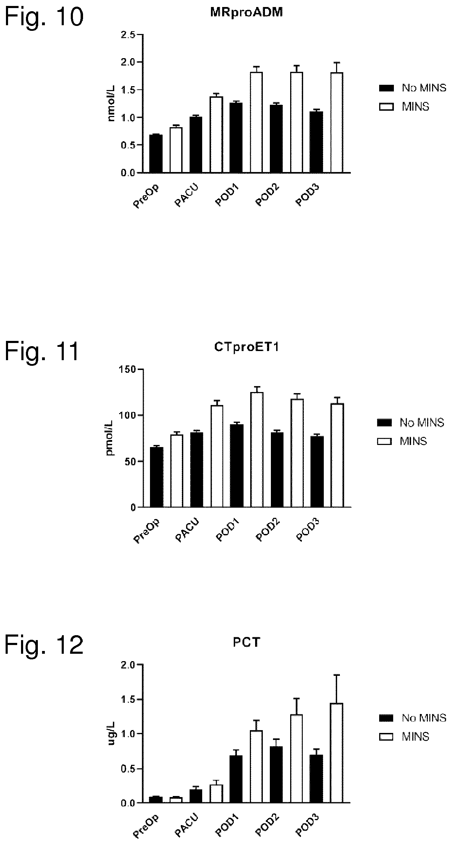

Figures 7-12 show the determined levels of the biomarkers in analogy to figures 1-6, wherein the patient population is divided in patients who did and did not develop a MINS.

### EXAMPLE

A multicenter, prospective cohort study in several Swedish hospitals was conducted. Subjects were recruited consecutively between April 2017 and October 2018. Patients aged 50 or older, undergoing elective abdominal surgery rated as major or major/complex by SORT, Surgical Outcome Risk Tool [Protopapa et al., British Journal of Surgery, 101(13), pp. 1774-1783. doi: 10.1002/bjs.9638, 2014], were eligible. The procedure also had to be performed under general anesthesia and require at least one overnight stay. Eligible patients were identified by screening daily patient lists in preoperative assessment clinics. Patients were informed of the study objectives, methods, expected benefits, potential dangers and the possibility of withdrawal at any time before consenting to participate. Informed written consent was obtained from all patients before being included. Participants could be included only once and patients were excluded if they were not able to give an informed consent or underwent one of the following types of abdominal surgery: transplantation, trauma, endocrine, vascular or endovascular. Included patients were excluded if the elective surgery was cancelled because the patient died, or if another non-elective surgery was performed prior to the elective one. The predefined study endpoint was MACCE (as defined above) within 30 days.

Five sample points were defined: preoperatively (<24 h prior to anesthesia), postoperatively (within 24 hours of surgery) and on days 1, 2 and 3 after surgery. At these times, an arterial or venous blood sample was obtained using an 8.5 ml EDTA Vacutainer®. Blood samples were sent to the local clinical chemistry laboratory where they were centrifuged, plasma was frozen in aliquots at -80 °C and stored until batch-analyses of plasma concentration of the biomarkers were performed. Simultaneously, an ECG was taken from each patient at each sampling point.

The occurrence of MACCE was reviewed on day 1, 2 and 3 through medical charts and at 30 days after surgery via a telephone interview and/or by consulting the patient's medical records. The incidence of MINS was calculated from analyzed cTnT plasma levels [according to Sessler and Khanna; Intensive Care Med (2018) 44:811-822, https://doi.org/10.1007/s00134-018-5224-7]. The mortality rate was assessed using the Swedish population register. If a patient was discharged, dropped out or died before all samples had been obtained, the patient was not excluded and the collected samples were analyzed unless the patient asked not to be included in the data analyses.

Copeptin, MR-proADM, CT-proET-1 and PCT were measured using a Thermo Scientific B·R·A·H·M·S Kryptor Compact plus according to the instructions of the manufacturer (B·R·A·H·M·S GmbH, Hennigsdorf, Germany). Cardiac troponin T (cTnT) and NT-proBNP were measured using a Cobas e 602/Cobas e 601/Cobas e 411 assay according to the instructions of the manufacturer (Roche Diagnostics, Mannheim, Germany).

Data analyses were performed using Graphpad PRISM 8. Data distribution was reviewed using D'Agostino & Pearson test. Comparisons between different groups were performed using Fisher's exact test and Kruskal Wallis test. P-values <0.05 were considered significant.

Patient- and clinical characteristics are presented in Table 1, wherein ASA stands for the physical status classification system according to the American Society of Anesthesiologists (ASA). 387 surgical patients were recruited and included in the analyses. It is noteworthy that only 43% of patients who suffered from MACCE were classified as high-risk patients (ASA III-IV), and only 38% were classified as having MINS.

**Table 1: Patient and clinical characteristics for patients with and without MACCE.**

| | **All** | **No MACCE** | **MACCE** |
|---|---|---|---|
| **No. of patients** | 387 | 350 | 37 (9.6%) |
| **Age median** | 70 | 70 | 72 |
| **Women** | 44% | 46% | 30% |
| **ASA I-II** | 265 | 243 | 21 |
| **ASA III-IV** | 116 (30%) | 100 (29%) | 16 (43%) |
| **MINS** | 94 (24%) | 80 (23%) | 14 (38%) |
| **Deceased** | 5 (1.3%) | 0 | 5 (13.5%) |

The collected data did not pass the D'Agostino-Pearson normality test, therefore non-parametric tests were used in the analyses. Comparison of how plasma levels of the measured biomarkers varied between patients affected, and not affected, by MACCE was performed using a Kruskal Wallis test. Figures 1-6 show plasma levels of the measured biomarkers copeptin, cTnT, NT-proBNP, MR-proADM, CT-proET-1 and PCT in patients with and without MACCE at all sampling points/times. In the figures, PreOp designates the preoperative sampling point, PACU (post anesthesia care unit) designates the postoperative sampling point, POD1 designates the sampling point one day after surgery, POD2 designates the sampling point two days after surgery and POD3 designates the sampling point three days after surgery.

As can be seen from figures 1-6, the biomarker levels of all measured biomarkers are increased in the group of patients who suffered a MACCE within 30 days from gastrointestinal surgery in comparison with the group of patients not suffering a MACCE.

The variation of the biomarker levels was also compared in patients with and without MINS using a Kruskal Wallis test. In analogy to figures 1-6, figures 7-12 show plasma levels of the measured biomarkers copeptin, cTnT, NT-proBNP, MR-proADM, CT-proET-1 and PCT in patients with and without MINS at all sampling points/times. Again, as can be seen from figures 7-12, the biomarker levels of all measured biomarkers are increased in the group of patients who suffered a MINS within 30 days from gastrointestinal surgery in comparison with the group of patients not suffering a MINS.

### SEQUENCES

SEQ ID NO:1 (amino acid sequence of pre-pro-AVP): 
1       
51      
101     
151     
SEQ ID NO:2 (amino acid sequence of pro-AVP): 
1       CYFQNCPRGG KRAMSDLELR QCLPCGPGGK GRCFGPSICC ADELGCFVGT
51      AEALRCQEEN YLPSPCQSGQ KACGSGGRCA AFGVCCNDES CVTEPECREG
101     FHRRARASDR SNATQLDGPA GALLLRLVQL AGAPEPFEPA QPDAY
SEQ ID NO:3 (amino acid sequence of CT-pre-proAVP or copeptin):
   1 ASDRSNATQL DGPAGALLLR LVQLAGAPEP FEPAQPDAY
SEQ ID NO:4 (amino acid sequence of Neurophysin II): 
1      AMSDLELRQC LPCGPGGKGR CFGPSICCAD ELGCFVGTAE ALRCQEENYL
51     PSPCQSGQKA CGSGGRCAAF GVCCNDESCV TEPECREGFH RRA
SEQ ID NO:5 (amino acid sequence of cTnT (Isoform-6)): 
1 
51 
101 
151 
201 
251 
SEQ ID NO:6 (amino acid sequence of cTnI): 
1       
51      
101     
151     
201     
SEQ ID NO:7 (amino acid sequence of TnC): 
1       MDDIYKAAVE QLTEEQKNEF KAAFDIFVLG AEDGCISTKE LGKVMRMLGQ
51      NPTPEELQEM IDEVDEDGSG TVDFDEFLVM MVRCMKDDSK GKSEEELSDL
101     FRMFDKNADG YIDLDELKIM LQATGETITE DDIEELMKDG DKNNDGRIDY
151     DEFLEFMKGV E
SEQ ID NO:8 (amino acid sequence of pre-pro-BNP): 
1 MDPQTAPSRA LLLLLFLHLA FLGGRSHPLG SPGSASDLET SGLQEQRNHL
51 QGKLSELQVE QTSLEPLQES PRPTGVWKSR EVATEGIRGH RKMVLYTLRA
101 PRSPKMVQGS GCFGRKMDRI SSSSGLGCKV LRRH
SEQ ID NO:9 (amino acid sequence of pro-BNP): 
1 HPLGSPGSAS DLETSGLQEQ RNHLQGKLSE LQVEQTSLEP LQESPRPTGV
51 WKSREVATEG IRGHRKMVLY TLRAPRSPKM VQGSGCFGRK MDRISSSSGL
101 GCKVLRRH
SEQ ID NO:10 (amino acid sequence of NT-pro-BNP): 
1       HPLGSPGSAS DLETSGLQEQ RNHLQGKLSE LQVEQTSLEP LQESPRPTGV
51      WKSREVATEG IRGHRKMVLY TLRAPR
SEQ ID NO:11 (amino acid sequence of BNP):
   1 SPKMVQGSGC FGRKMDRISS SSGLGCKVLR RH
SEQ ID NO:12 (amino acid sequence of pre-pro-ADM): 
1       MKLVSVALMY LGSLAFLGAD TARLDVASEF RKKWNKWALS RGKRELRMSS
51      SYPTGLADVK AGPAQTLIRP QDMKGASRSP EDSSPDAARI RVKRYRQSMN
101     NFQGLRSFGC RFGTCTVQKL AHQIYQFTDK DKDNVAPRSK ISPQGYGRRR
151     RRSLPEAGPG RTLVSSKPQA HGAPAPPSGS APHFL
SEQ ID NO:13 (amino acid sequence of pro-ADM): 
1 ARLDVASEFR KKWNKWALSR GKRELRMSSS YPTGLADVKA GPAQTLIRPQ
51 DMKGASRSPE DSSPDAARIR VKRYRQSMNN FQGLRSFGCR FGTCTVQKLA
101 HQIYQFTDKD KDNVAPRSKI SPQGYGRRRR RSLPEAGPGR TLVSSKPQAH
151 GAPAPPSGSA PHFL
SEQ ID NO:14 (amino acid sequence of MR-pro-ADM):
   1 ELRMSSSYPT GLADVKAGPA QTLIRPQDMK GASRSPEDSS PDAARIRV
SEQ ID NO:15 (amino acid sequence of pre-pro-ET-1): 
1       MDYLLMIFSL LFVACQGAPE TAVLGAELSA VGENGGEKPT PSPPWRLRRS
51      KRCSCSSLMD KECVYFCHLD IIWVNTPEHV VPYGLGSPRS KRALENLLPT
101     KATDRENRCQ CASQKDKKCW NFCQAGKELR AEDIMEKDWN NHKKGKDCSK
151     LGKKCIYQQL VRGRKIRRSS EEHLRQTRSE TMRNSVKSSF HDPKLKGKPS
201     RERYVTHNRA HW
SEQ ID NO:16 (amino acid sequence of pro-ET-1): 
1 APETAVLGAE LSAVGENGGE KPTPSPPWRL RRSKRCSCSS LMDKECVYFC
51 HLDIIWVNTP EHVVPYGLGS PRSKRALENL LPTKATDREN RCQCASQKDK
101 KCWNFCQAGK ELRAEDIMEK DWNNHKKGKD CSKLGKKCIY QQLVRGRKIR
151 RSSEEHLRQT RSETMRNSVK SSFHDPKLKG KPSRERYVTH NRAHW
SEQ ID NO:17 (amino acid sequence of ET-1):
   1 CSCSSLMDKE CVYFCHLDII W
SEQ ID NO:18 (amino acid sequence of CT-pro-ET-1):
   1 RSSEEHLRQT RSETMRNSVK SSFHDPKLKG KPSRERYVTH NRAHW
SEQ ID NO:19 (amino acid sequence of Big-ET-1):
   1 CSCSSLMDKE CVYFCHLDII WVNTPEHVVP YGLGSPRS
SEQ ID NO:20 (amino acid sequence of PCT): 
1 APFRSALESS PADPATLSED EARLLLAALV QDYVQMKASE LEQEQEREGS
51 SLDSPRSKRC GNLSTCMLGT YTQDFNKFHT FPQTAIGVGA PGKKRDMSSD
101 LERDHRPHVS MPQNAN
SEQ ID NO:21 (amino acid sequence of pre-pro-ANP (human)): 
1 
51 
101 
151 
SEQ ID NO:22 (amino acid sequence of pro-ANP (human)): 
1       NPMYNAVSNA DLMDFKNLLD HLEEKMPLED EVVPPQVLSE PNEEAGAALS
51      PLPEVPPWTG EVSPAQRDGG ALGRGPWDSS DRSALLKSKL RALLTAPRSL
101     RRSSCFGGRM DRIGAQSGLG CNSFRY
SEQ ID NO:23 (amino acid sequence of NT-proANP): 
1     NPMYNAVSNA DLMDFKNLLD HLEEKMPLED EVVPPQVLSE PNEEAGAALS
51    PLPEVPPWTG EVSPAQRDGG ALGRGPWDSS DRSALLKSKL RALLTAPR
SEQ ID NO:24 (MR-proANP = amino acid sequence of amino acids 53-90 of proANP):
   1 PEVPPWT GEVSPAQRDG GALGRGPWDS SDRSALLKSK L
SEQ ID NO:25 (Myoglobin (human)) 
1 MGLSDGEWQL VLNVWGKVEA DIPGHGQEVL IRLFKGHPET LEKFDKFKHL
51 KSEDEMKASE DLKKHGATVL TALGGILKKK GHHEAEIKPL AQSHATKHKI
101 PVKYLEFISE CIIQVLQSKH PGDFGADAQG AMNKALELFR KDMASNYKEL
151 GFQG
SEQ ID NO:26 (Creatine kinase (human)) 
1 MPFSNSHNAL KLRFPAEDEF PDLSAHNNHM AKVLTPELYA ELRAKSTPSG
51 FTLDDVIQTG VDNPGHPYIM TVGCVAGDEE SYEVFKDLFD PIIEDRHGGY
101 KPSDEHKTDL NPDNLQGGDD LDPNYVLSSR VRTGRSIRGF CLPPHCSRGE
151 RRAIEKLAVE ALSSLDGDLA GRYYALKSMT EAEQQQLIDD HFLFDKPVSP
201 LLLASGMARD WPDARGIWHN DNKTFLVWVN EEDHLRVISM QKGGNMKEVF
251 TRFCTGLTQI ETLFKSKDYE FMWNPHLGYI LTCPSNLGTG LRAGVHIKLP
301 NLGKHEKFSE VLKRLRLQKR GTGGVDTAAV GGVFDVSNAD RLGFSEVELV
351 QMVVDGVKLL IEMEQRLEQG QAIDDLMPAQ K
SEQ ID NO:27 (C reactive Protein (human))

1 MEKLLCFLVL TSLSHAFGQT DMSRKAFVFP KESDTSYVSL KAPLTKPLKA
51 FTVCLHFYTE LSSTRGYSIF SYATKRQDNE ILIFWSKDIG YSFTVGGSEI
101 LFEVPEVTVA PVHICTSWES ASGIVEFWVD GKPRVRKSLK KGYTVGAEAS
151 IILGQEQDSF GGNFEGSQSL VGDIGNVNMW DFVLSPDEIN TIYLGGPFSP
201 NVLNWRALKY EVQGEVFTKP QLWP

## Claims

1. A method for at least one of prognosis, risk assessment and diagnosis of a major adverse cardio- or cerebrovascular event (MACCE) in a patient who underwent gastrointestinal surgery, the method comprising the steps of:
i) providing a sample of a bodily fluid from said patient,
ii) determining in said sample the level of a biomarker selected from the group consisting of copeptin, troponin and brain natriuretic peptide (BNP),
iii) determining at least one additional parameter of said patient,
iv) combining the biomarker level determined in step ii) and the additional parameter determined in step iii) into a combined assessment, and
v) correlating the combined assessment to said at least one of prognosis, risk assessment and diagnosis of a MACCE in said patient.

2. The method according to claim 1,
wherein the additional parameter is selected from the group consisting of the level of at least one additional biomarker and a clinical parameter of said patient.

3. The method according to claim 2,
wherein the additional biomarker is selected from the group consisting of copeptin, troponin, BNP, mid-regional proadrenomedullin (MR-proADM), C-terminal proendothelin-1 (CT-proET-1), procalcitonin (PCT), MR-proANP (mid-regional pro atrial natriuretic peptide), creatinine kinase, creatine kinase-MB, myoglobin, lactate and CRP (C-reactive protein).

4. The method according to claim 2 or 3,
wherein the levels of the biomarkers copeptin, troponin and brain natriuretic peptide (BNP) and, optionally, at least one of MR-proADM, CT-proET-1 and PCT are determined and combined into the combined assessment.

5. The method according to any one of claims 2 to 4,
wherein the clinical parameter is selected from the group consisting of age, abnormal ECG, especially pathological Q-waves, LBBB, ST-elevation, ST-depression, T-wave inversion, intraoperative hypotension, intraoperative tachycardia, intraoperative bradycardia, hyperlipidaemia, smoking status, anaemia, functional capacities (METs), red-blood cell transfusion, arrhythmias, rhythm other than sinus, duration of the gastrointestinal surgery and operation size, patient history and liver disease.

6. The method according to any one of the preceding claims,
wherein the level of at least one of the biomarkers is determined by determining the level of at least one of a precursor, a precursor fragment and a fragment of the biomarker.

7. The method according to claim any one of the preceding claims,
wherein determining the level of troponin comprises determining the level of the subunit cardiac troponin T (cTnT), preferably isoform 6 of cTnT or a homologous peptide with at least 75% amino acid sequence identity with isoform 6 of cTnT.

8. The method according to any one of any one of the preceding claims,
wherein determining the level of BNP comprises determining the level of precursor fragment NT-proBNP.

9. The method according to any one of the preceding claims,
wherein the patient is at least 50 years old.

10. The method according to any one of the preceding claims,
wherein the patient is treated with analgesics or pain treatment.

11. The method according to any one of the preceding claims,
wherein the patient spent at least one day in a hospital after undergoing the gastrointestinal surgery.

12. The method according to any one of the preceding claims,
wherein the gastrointestinal surgery is one of laparoscopic and open and is selected from the group consisting of
- stomach surgery,
- small intestine surgery, in particular duodenum surgery,
- large intestine surgery, in particular rectum surgery,
- reproductive system surgery, in particular hysterectomy or salpingoophorectomy,
- kidney surgery, in particular nephrectomy,
- urinary bladder surgery, in particular cystectomy,
- gallbladder surgery, in particular cholecystectomy, and
- surgery of gastrointestinal cysts.

13. The method according to any one of the preceding claims,
wherein the patient is free from cardiovascular comorbidities.

14. The method according to any one of the preceding claims,
wherein the patient is excluded if an exclusion condition is present in the patient, the exclusion condition being selected from the group consisting of organ transplantation, traumatic injury, endocrine disease, endocrine surgery, vascular disease, vascular surgery, endovascular disease, endovascular surgery, acute coronary syndrome (ACS), heart failure, decompensated congestive heart failure, aortic stenosis, reduced left ventricular ejection fraction (LVEF), and circulatory shock.

15. The method according to any one of the preceding claims,
wherein the sample has been taken from the patient no more than 24 hours before the gastrointestinal surgery, no more than 24 hours after the gastrointestinal surgery, on day one after the gastrointestinal surgery, on day two after the gastrointestinal surgery or on day three after the gastrointestinal surgery.

16. The method according to any one of the preceding claims,
wherein the method comprises providing of from two to five samples from the patient, wherein the samples are taken at different times before and/or after the gastrointestinal surgery, and wherein steps ii), iv) and v) are practiced on all said samples.

17. The method according to claim 16,
wherein the different times said of from two to five samples are taken are selected from the group consisting of no more than 24 hours before the gastrointestinal surgery, no more than 24 hours after the gastrointestinal surgery, day one after the gastrointestinal surgery, day two after the gastrointestinal surgery and day three after the gastrointestinal surgery.

18. The method according to any one of claims 16-17,
wherein the levels of said biomarkers in the samples taken at different times are determined and then compared, and wherein the differences in the levels of the biomarkers at different times are combined into the combined assessment.

19. The method according to any one of the preceding claims,
wherein a biomarker level determined to be above a specific threshold value is indicative of a MACCE in the patient.

20. The method according to claim 19,
wherein the specific threshold value is selected from the group consisting of
- 25 pmol/L, preferably 75 pmol/L, more preferably 125 pmol/L, even more preferably 175 pmol/L and most preferably 225 pmol/L for copeptin,
- 15 ng/L, preferably 20 ng/L, more preferably 25 ng/L, even more preferably 30 ng/L and most preferably 35 ng/L, 45 ng/L or 65 ng/L for cTnT,
- 500 ng/L, preferably 900 ng/L, more preferably 1300 ng/L, even more preferably 1700 ng/L and most preferably 2100 ng/L or 2800 ng/L for NT-proBNP,
- 0.8 nmol/L or 1.0 nmol/L, preferably 1.25 nmol/L, more preferably 1.5 nmol/L, even more preferably 1.75 nmol/L and most preferably 2.0 nmol/L or 2.4 nmol/L for MR-proADM,
- 80 pmol/L, preferably 90 pmol/L, more preferably 100 pmol/L, even more preferably 110 pmol/L and most preferably 120 pmol/L for CT-proET-1, and
- 0.5 *µ*g/L, preferably 1.0 *µ*g/L, more preferably 1.5 *µ*g/L, even more preferably 2.0 *µ*g/L and most preferably 2.5 *µ*g/L or 3.0 *µ*g/L for PCT.

21. The method according to any one of the preceding claims,
wherein the method comprises correlating the combined assessment to the risk of a MACCE in the patient within at least one of 30 days and 12 months after the gastrointestinal surgery.

22. The method according to any one of claims 17-18,
wherein the prognosis comprises the risk of mortality within at least one of 30 days and 12 months after the gastrointestinal surgery.

23. The method according to any one of the preceding claims,
wherein the MACCE is a myocardial injury after noncardiac surgery (MINS) and the method is a method for the diagnosis of a MINS in a patient who underwent gastrointestinal surgery.

24. The method according to claim 20,
wherein a biomarker level determined to be above a specific threshold value is indicative of a MINS in the patient.

25. The method according to claim 22,
wherein the specific threshold value is selected from the group consisting of
- 25 pmol/L, preferably 50 pmol/L or 75 pmol/L, more preferably 125 pmol/L, even more preferably 175 pmol/L and most preferably 225 pmol/L for copeptin,
- 10 ng/L, preferably 20 ng/L, more preferably 30 ng/L, even more preferably 40 ng/L and most preferably 50 ng/L or 60 ng/L for cTnT,
- 250 ng/L or 500 ng/L, preferably 750 ng/L, more preferably 1250 ng/L or 1500 ng/L, even more preferably 1750 ng/L and most preferably 2250 ng/L for NT-proBNP,
- 0.8 nmol/L or 1.0 nmol/L, preferably 1.25 nmol/L, more preferably 1.5 nmol/L, even more preferably 1.75 nmol/L and most preferably 2.0 nmol/L for MR-proADM,
- 65 pmol/L or 75 pmol/L or 80 pmol/L, preferably 90 pmol/L, more preferably 100 pmol/L, even more preferably 110 pmol/L and most preferably 120 pmol/L for CT-proET-1, and
- 0.2 *µ*g/L or 0.5 *µ*g/L, preferably 0.75 *µ*g/L or 0.8 *µ*g/L, more preferably 1.0 *µ*g/L, even more preferably 1.25 *µ*g/L and most preferably 1.5 *µ*g/L for PCT.

26. The method according to any one of claims 16-25,
wherein the determined biomarker levels taken at different times are evaluated using different threshold values.

27. The method according to any one of claims 16-26,
wherein the determined biomarker levels taken at different times are differently weighted.

28. The method according to any one of claims 16-27,
wherein a relative change in the biomarker level between two samples taken at different times is combined into the combined assessment.

29. The method according to any one of the preceding claims,
wherein a ratio between the levels of at least two biomarkers in the sample is determined and said ratio is combined into the combined assessment, wherein the biomarkers for determining the ratio are preferably selected from copeptin/troponin, copeptin/BNP and BNP/troponin.

30. The method according to any one of the preceding claims,
wherein step iv) comprises providing reference data for the determined biomarkers and at least one value selected from the group consisting of
- the determined level of at least one of the biomarkers,
- the differences in the level of at least one of the biomarkers taken at different times,
- the relative change in the levels of one of said biomarkers determined in two samples taken at different times
- the ratio between the levels of at least two biomarkers either taken at the same time or taken at different times and
- the relative change of the ratios of at least two biomarkers determined in at least two samples taken at different times
is compared to the reference data, wherein the difference of the value as determined and the reference data is computed, and wherein the computed difference is preferably expressed in the form of a score, especially as a numerical value.

31. The method according to claim 30,
wherein at least two different scores are determined and the combined assessment comprises a combined score determined from said at least two different scores indicative of the presence or absence of a MACCE in the patient.

32. The method according to any one of claims 30-31,
wherein the reference data pertains to patients who underwent gastrointestinal surgery and who did and/or did not have a MACCE or MINS.

33. Kit for carrying out the method according to any one of the preceding claims, comprising:
at least one detection reagent for determining the level of at least one of the biomarkers copeptin, troponin and brain natriuretic peptide (BNP) in a sample of a bodily fluid from a patient, and
reference data from patients who underwent gastrointestinal surgery, particularly reference levels, corresponding to at least one of copeptin, troponin and BNP levels, wherein said reference data is preferably stored on a computer-readable medium and/or employed in the form of computer-executable code configured for comparing the determined level of at least one of copeptin, troponin and BNP to said reference data.

34. The kit according to claim 33,
wherein the at least one detection reagent comprises reagents for determining the levels of the biomarkers copeptin, troponin and brain natriuretic peptide (BNP) and, optionally, at least one of MR-proADM, CT-proET-1 and PCT in a sample of a bodily fluid from said patient, and wherein the reference data comprises data corresponding to said biomarkers in patients who underwent gastrointestinal surgery.

35. Computer, comprising means for running computer-executable code, with the computer-executable code being configured to carry out steps iv) and/or v) of the method of any of claims 1-32.

36. Computer according to claim 35,
wherein the computer is connected to an assay system, the assay system being configured to carry out step ii) of the method of any of claims 1-32.

37. Computer program product comprising a set of computer instructions stored on at least one computer-readable medium, said set of computer instructions further comprising instructions executable by one or more processors to carry out steps iv) and/or v) of the method of any of claims 1-32.
